# EUROPEAN PATENT SPECIFICATION

(11) **EP 3 481 806 B1**
(45) Date of publication and mention of the grant of the patent: **07.06.2023**
(21) Application number: 17735589.8
(22) Date of filing: 10.07.2017
(51) Int. Cl.: C07D 215/44, A61K 31/4706, A61P 35/00

(54) **4-ANILINO-QUINOLINE COMPOUNDS AS ANTI-CANCER AGENTS**
4-ANILINO-CHINOLINVERBINDUNGEN ALS ANTIKREBSMITTEL
COMPOSÉS DE 4-ANILINO-QUINOLÉINE SUBSTITUÉS COMME AGENTS ANTICANCEREUX

(30) Priority: 08.07.2016 EP 16305862
(43) Date of publication of application: 15.05.2019
(73) Proprietor: INSERM (Institut National de la Santé et de la Recherche Médicale), 75013 Paris (FR); Université Côte d'Azur, 06108 Nice Cedex 2 (FR); Centre National de la Recherche Scientifique CNRS, 75016 Paris (FR); CHU de Nice, 06000 Nice (FR)
(72) Inventor: PASSERON, Thierry, 06204 Nice (FR); BENHIDA, Rachid, 06108 NICE cedex 02 (FR); DAO, Pascal, 06700 Saint Laurent du Var (FR); DE DONATIS Gian Marco, Cambridge, CB2 9EU (GB); MARTIN, Anthony, 34090 Montpellier (FR)
(74) Representative: Vossius & Partner Patentanwälte Rechtsanwälte mbB
(86) International application number: PCT/EP2017/067306
(87) International publication number: WO 2018/007648

(56) References cited:
- WO-A2-2008/089307
- CN-A- 102 249 997
- US-A- 3 075 981
- DAKSHANAMURTHY SIVANESAN ET AL: "In-silico fragment-based identification of novel angiogenesis inhibitors", BIOORGANIC & MEDICINAL CHEMISTRY LETTERS, vol. 17, no. 16, 15 July 2007 (2007-07-15) , pages 4551-4556, XP029094771, ISSN: 0960-894X, DOI: 10.1016/J.BMCL.2007.05.104
- GOZALBES RAFAEL ET AL: "Hit identification of novel heparanase inhibitors by structure- and ligand-based approaches", BIOORGANIC & MEDICINAL CHEMISTRY, PERGAMON, GB, vol. 21, no. 7, 31 January 2013 (2013-01-31), pages 1944-1951, XP029002574, ISSN: 0968-0896, DOI: 10.1016/J.BMC.2013.01.033
- Dan Liu: "Molecules | Free Full-Text | Synthesis and Anti-Tumor Activities of 4-Anilinoquinoline Derivatives", molecules, 23 December 2015 (2015-12-23), pages 1-8, XP055298743, Retrieved from the Internet: URL:http://www.mdpi.com/1420-3049/21/1/21 [retrieved on 2016-08-30]
- Elaine S Coimbra ET AL: "Available free online at www Amodiaquine analogs. Synthesis and anti-leishmanial activity", Mediterranean Journal of Chemistry, 1 January 2011 (2011-01-01), pages 106-113, XP055298745, Retrieved from the Internet: URL:http://www.medjchem.com/images/stories /V1N3/mjcsouza2.pdf
- HE L ET AL: "Design of antineoplastic agents based on the '2-phenylnaphthalene-type' structural pattern-synthesis and biological activity studies of 11H-indolo[3.2-c]quinoline derivatives", EUROPEAN JOURNAL OF MEDICINAL CHEMI, EDITIONS SCIENTIFIQUE ELSEVIER, PARIS, FR, vol. 38, no. 1, 1 January 2003 (2003-01-01), pages 101-107, XP004410831, ISSN: 0223-5234, DOI: 10.1016/S0223-5234(02)01420-4
- BEAUCHARD A ET AL: "Synthesis of original thiazoloindolo[3,2-c]quinoline and novel 8-N-substituted-11H-indolo[3,2-c]quinoline derivatives from benzotriazoles. Part I", TETRAHEDRON, ELSEVIER SCIENCE PUBLISHERS, AMSTERDAM, NL, vol. 62, no. 8, 20 February 2006 (2006-02-20), pages 1895-1903, XP025001577, ISSN: 0040-4020, DOI: 10.1016/J.TET.2005.09.153 [retrieved on 2006-02-20]
- CAROLINE MEYERS ET AL: "Auto-Tandem Catalysis: Synthesis of Substituted 11H-Indolo[3,2-c]quinolinesvia Palladium-Catalyzed Intermolecular C-N and Intramolecular C-C Bond Formation", ADVANCED SYNTHESIS & CATALYSIS, vol. 350, no. 3, 22 February 2008 (2008-02-22), pages 465-470, XP055403827, DE ISSN: 1615-4150, DOI: 10.1002/adsc.200700328
- ROSARIO SÁNCHEZ-MARTÍN ET AL: "Symmetrical Bis-Quinolinium Compounds: New Human Choline Kinase Inhibitors with Antiproliferative Activity against the HT-29 Cell Line", JOURNAL OF MEDICINAL CHEMISTRY, vol. 48, no. 9, 1 May 2005 (2005-05-01), pages 3354-3363, XP055011210, ISSN: 0022-2623, DOI: 10.1021/jm049061o
- XIANG PU ET AL: "5-methoxyquinoline derivatives as a new class of EZH2 inhibitors", MOLECULES: A JOURNAL OF SYNTHETIC ORGANIC AND NATURAL PRODUCT CHEMI, M D P I AG, CH, vol. 20, no. 5, 1 January 2015 (2015-01-01), pages 7620-7636, XP009188456, ISSN: 1420-3049
- DENNY W A: "The 4-anilinoquinazoline class of inhibitors of the erbB family of receptor tyrosine kinases", IL FARMACO, ELSEVIER FRANCE * EDITIONS SCIENTIFIQUES ET MEDICALES, FR, vol. 56, 1 January 2001 (2001-01-01), pages 51-56, XP003015967, ISSN: 0014-827X, DOI: 10.1016/S0014-827X(01)01026-6

## Description

### FIELD OF THE INVENTION:

The invention relates to novel compounds active for the treatment of cancer, as further defined in the claims.

### BACKGROUND OF THE INVENTION:

Cutaneous melanoma deriving from the transformation of melanocytes is one of the most lethal cancers among young adults. Its incidence has increased at a dramatic rate during the last decades. Melanoma has a high capability of invasion and rapid metastasis to other organs.

Immune-checkpoint blockades targeting cytotoxic T-lymphocyte-associated protein 4 (CTLA-4), and more recently Programmed Death 1 (PD1) and Programmed Death-Ligand 1 (PD-L1), are recent and major breakthroughs in cancer therapy. Initially developed to treat metastatic melanomas, antibodies against those targets significantly increase overall patient survival and are now being evaluated to treat other solid cancers such as those of the kidney, prostate, colon, and lung. Although anti-PD1 antibodies have shown better results than anti-CTLA-4 antibodies, the response rate remains low (10% to 57%), depending on the cancer type and the treatment combinations. The combination of anti-PD1 and anti-CTLA-4 antibodies to treat melanoma has given the best complete response rate so far, 11.5%, but is associated with an almost 70% rate of grade 3 or grade 4 side effects. Few patients therefore benefit from those approaches, and no predictive factor for the response has yet been identified. Accumulating evidence suggests that interferon gamma (IFN-γ) plays a key role in the response to anti-PD1 treatment (1-3). A meta-analysis of all the immune-based approaches to melanoma treatment (including anti-PD1) showed that patients with vitiligoid depigmentation have significantly better rates of progression-free survival and overall survival compared with other patients (4). Furthermore, patients with vitiligo have a threefold lesser risk of developing melanoma (5). An increasing number of data indicate that the IPN-γ/CXCL10 pathway, which is involved in the vitiligoid depigmentation process, plays a key role in determining melanoma risk (6). Thus, the IFN-γ response is implicated as a key factor facilitating the checkpoint-blockade treatment approaches. Inventors of the instant application recently showed that the inhibition of the non-canonical NF-kB pathway, as well as that of the upstream NF-kB-inducing kinase (NIK), restores a senescence program in melanoma cells through decreased EZH2 transcription and significantly reduces tumor growth (7). There are increasing evidences that cellular senescence can trigger or potentiate tumor immune surveillance (8).

CN 102249997 A relates to certain 4-substituted phenylaminoquinoline compounds having antitumor activity. Dakshanamurthy S et al., Bioorg Med Chem Lett, 2007, 17(16):4551-6 relates to the *in-silico* fragment-based identification of novel angiogenesis inhibitors. Gozalbes R et la., Bioorg Med Chem, 2013, 21(7):1944-51 relates to the hit identification of novel heparanase inhibitors by structure- and ligand-based approaches. Liu D et al., Molecules, 2015, 21(1):E21 relates to the synthesis and anti-tumor activities of certain 4-anilinoquinoline derivatives.

The instant invention provides new NIK inhibitors that decrease EZH2 at the transcriptional level and induce the production of an IFN-γ response by the treated cell. These NIK inhibitors reduce the size of subcutaneous tumors without showing any specific toxicity, and when combined with anti-PD1 treatment lead to a dramatic reduction in tumor size with complete regression in some cases. Those effects are associated with a marked increase in the numbers and activation of macrophages, dendritic cells, and T-cells within the treated tumors.

### SUMMARY OF THE INVENTION:

The present invention relates to the following aspects:
In aspect [1], the invention provides a compound of general formula (I): 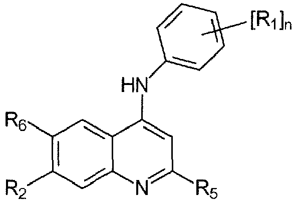 wherein
   R₁ is selected from H, alkyl, Halo, OH, O-alkyl, NH₂, NH-alkyl, N-(alkyl)₂, S-alkyl, CF₃, OCF₃, OCF₂H and O(CH₂)ₙ₁O(CH₂)ₙ₂CH₃, n₁ being 1 to 4 and n₂ being 0 to 3, Halo being selected from Cl, F, Br and I;
   n is 1 or 2,
   when n is 2, then it is also possible for R₁ to form with the phenyl group at the meta and para positions a dioxolane group or a 1,4 dioxane group,
   R₂ is selected from Halo and NR₃R₄, Halo being defined as previously,
   R₃ is H and R₄ is phenyl optionally substituted with one or more substituents selected from OH, O-alkyl and Halo,
   or R₃ and R₄ together form a (CH₂)₂O(CH₂)₂ chain, that is to say that NR₃R₄ forms a morpholino group 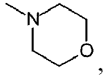
   R₅ is selected from H, cyclic radical, O-alkyl, O-(CH₂)ₙ₁-(cyclic radical), (CH₂)ₙ₁-(cyclic radical), n₁ being defined as previously, said cyclic radical being chosen from 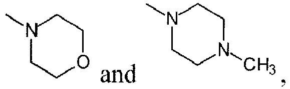
   R₆ is selected from H and Halo,
   with the proviso that when R₂ is Halo or R₆ is Halo then R₅ is not H,
   or a pharmaceutically acceptable salt and/or optical isomer, tautomer, solvate or isotopic variation thereof.
In aspect [2], the invention provides a compound according to aspect [1], which is of general formula (2): 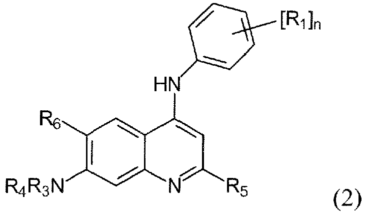
   wherein R₁, R₃, R₄, R₅, R₆ and n are as defined in aspect [1],
   or a pharmaceutically acceptable salt and/or optical isomer, tautomer, solvate or isotopic variation thereof.
In aspect [3], the invention provides a compound according to aspect [1], which is of general formula (3): 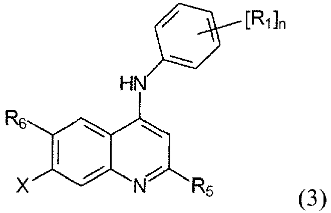
   wherein R₁, R₅, R₆, and n are as defined in aspect [1] and X is a Halo chosen from Cl, F, Br and I, and with the proviso that R₅ is not H,
   or a pharmaceutically acceptable salt and/or optical isomer, tautomer, solvate or isotopic variation thereof.
In aspect [4], the invention provides a compound according to any one of the above aspects [1] to [3], wherein R₁ is selected from methyl (CH₃), fluoro (F), chloro (Cl), hydroxy (OH), methoxy (OCH₃), CF₃, OCF₃, OCF₂H and O(CH₂)₂OCH₃, wherein when n is 2 then the substituents R₁ are identical or different.
In aspect [5], the invention provides a compound according to any one of the above aspects [1] to [3], wherein when n is 2 then the two R₁ form with the phenyl group at the meta and para positions a dioxolane group or a 1,4 dioxane group.
In aspect [6], the invention provides a compound according to any one of above aspects [1], [2] and [4], wherein R₃ is H and R₄ is phenyl unsubstituted or substituted with one or two groups identical or different group selected from OH, OCH₃ and Halo, preferably chloro.
In aspect [7], the invention provides a compound according to any one of the above aspects [1], [2] and [4], wherein R₃ and R₄ together form a (CH₂)₂O(CH₂)₂ chain, that is to say that NR₃R₄ forms a morpholino group.
In aspect [8], the invention provides a compound according to aspect [1], which is selected from:
   - *N*-(3,5-difluorophenyl)-7-morpholinoquinolin-4-amine,
   - *N*-(*p*-tolyl)-7-morpholinoquinolin-4-amine,
   - *N*-(*o*-tolyl)-7-morpholinoquinolin-4-amine,
   - *N*-(4-methoxyphenyl)-7-morpholinoquinolin-4-amine,
   - *N*-(3-methoxyphenyl)-7-morpholinoquinolin-4-amine,
   - *N*-(3-(trifluoromethyl)phenyl)-7-morpholinoquinolin-4-amine,
   - *N⁴,N⁷*-bis(4-methoxyphenyl)quinolin-4,7-diamine,
   - *N⁴,N*⁷-bis(3-methoxyphenyl)quinolin-4,7-diamine,
   - *N⁴*-(3,5-difluorophenyl)-*N⁷*-(4-methoxyphenyl)quinolin-4,7-diamine,
   - *N⁴*-(3,5-difluorophenyl)-*N⁷*-(3-chloro-4-hydroxyphenyl)quinolin-4,7-diamine,
   - *N⁴*-(*p*-tolyl)-*N⁷*-(3-methoxyphenyl)quinolin-4,7-diamine,
   - *N⁴*-(*p*-tolyl)-*N⁷*-(4-methoxyphenyl)quinolin-4,7-diamine,
   - *N⁴*-(3-(trifluoromethyl)phenyl)-*N⁷*-(4-methoxyphenyl)quinolin-4,7-diamine,
   - *N⁴*-(*o*-tolyl)-*N⁷*-(3 -methoxyphenyt)quinolin-4,7-diamine,
   - *N⁴*-(4-methoxyphenyl)-*N⁷*-(3-methoxyphenyl)quinolin-4,7-diamine,
   - *N⁴*-(3-methoxyphenyl)-*N⁷*-(4-methoxyphenyl)quinolin-4,7-diamine.
   - *N⁴*-(3,5-difluorophenyl)-*N⁷*-(3-methoxyphenyl)quinolin-4,7-diamine,
   - *N*-(4-methoxyphenyl)-7-chloro-2-methoxy-quinolin-4-amine,
   - *N*-(3,4-dimethoxyphenyl)-7-chloro-2-morpholino-quinolin-4-amine,
   - *N*-(3,4-dimethoxyphenyl)-7-chloro-2-(4-methylpiperazin-1-yl)-quinolin-4-amine,
   - *N*-(4-methoxyphenyl)-7-chloro-2-morpholino-quinolin-4-amine,
   - *N*-(4-methoxyphenyl)-7-chloro-2-(4-methylpiperazin-1 -yl)-quinolin-4-amine,
   - *N*-(3,4-dimethoxyphenyl)-7-chloro-2-(2-morpholinoethoxy)quinolin-4-amine and
   - *N*-(3,4-dimethoxyphenyl)-7-chloro-2-methoxy-quinolin-4-amine;
   or a pharmaceutically acceptable salt and/or optical isomer, tautomer, solvate or isotopic variation thereof.
In aspect [9], the invention provides a compound according to any one of the above aspects [1] to [8] for use in the treatment of cancer, said cancer being preferably selected from melanoma, bladder, kidney, prostate, colon, lung, breast and blood cancer.
In aspect [10], the invention provides a pharmaceutical composition as a combined preparation for simultaneous, separate or sequential use in the treatment of cancer, comprising:
   - a compound of general formula (I) 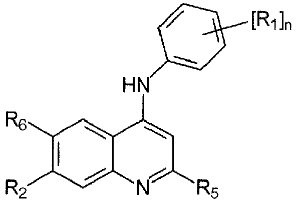 wherein
      R₁ is selected from H, alkyl, Halo, OH, O-alkyl, NH₂, NH-alkyl, N-(alkyl)₂, S-alkyl, CF₃, OCF₃, OCF₂H and O(CH₂)ₙ₁O(CH₂)ₙ₂CH₃, n₁ being 1 to 4 and n₂ being 0 to 3, Halo being selected from C1, F, Br and I;
      n is 1 or 2;
      when n is 2, then it is also possible for R₁ to form with the phenyl group at the meta and para positions a dioxolane group or a 1,4 dioxane group,
      R₂ is selected from H, Halo and NR₃R₄; Halo being defined as previously,
      R₃ is H and R₄ is phenyl optionally substituted with one or more substituents selected from OH, O-alkyl and Halo, or R₃ and R₄ together form a (CH₂)₂O(CH₂)₂ chain,
      R₅ is selected from H, cyclic radical, O-alkyl, O-(CH₂)ₙ₁-(cyclic radical), (CH₂)ₙ₁-(cyclic radical), n₁ being defined as previously, said cyclic radical being chosen from 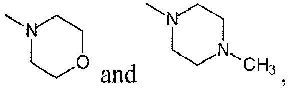
      R₆ is selected from H and Halo,
      or a pharmaceutically acceptable salt and/or optical isomer, tautomer, solvate or isotopic variation thereof,
   - at least one antibody selected from an anti-PD1 antibody, an anti-CTLA4 antibody, an anti-PD-L1 antibody and a mixture of two or more thereof, and
   - optionally at least one pharmaceutically acceptable carrier.
In aspect [11], the invention provides the pharmaceutical composition for use according to aspect [10], wherein compound (I) is selected from:
   - *N*-(3,5-difluorophenyl)-7-chloroquinolin-4-amine,
   - *N*-(4-methylphenyl)-7-chloroquinolin-4-amine,
   - *N-*(3-trifluoromethylphenyl)-7-chloroquinolin-4-amine,
   - *N*-(2-methylphenyl)-7-chloroquinolin-4-amine,
   - *N*-(4-methoxyphenyl)-7-chloroquinolin-4-amine,
   - *N*-(3-methoxyphenyl)-7-chloroquinolin-4-amine,
   - *N*-(4-hydroxyphenyl)-7-chloroquinolin-4-amine,
   - *N*-(4-hydroxy-3-chlorophenyl)-7-chloroquinolin-4-amine,
   - *N*-(4-trifluoromethylphenyl)-7-chloroquinolin-4-amine,
   - *N*-(3,5-difluorophenyl)-7-morpholinoquinolin-4-amine,
   - *N*-(4-(difluoromethoxy)phenyl)-7-chloro-quinolin-4-amine,
   - *N*-(4-methoxy-3-(2-methoxyethoxy)phenyl)-7-chloro-quinolin-4-amine,
   - *N*-(4-trifluoromethoxyphenyl)-6-chloroquinolin-4-amine,
   - *N*-(2,4-dimethoxyphenyl)-6-chloroquinolin-4-amine,
   - *N*-(3,4-dimethoxyphenyl)-7-chloroquinolin-4-amine,
   - *N*-(2,4-dimethoxyphenyl)-7-chloroquinolin-4-amine,
   - *N*-(*p*-tolyl)-7-morpholinoquinolin-4-amine,
   - *N*-(*o*-tolyl)-7-morpholinoquinolin-4-amine,
   - *N*-(4-methoxyphenyl)-7-morpholinoquinolin-4-amine,
   - *N*-(3-methoxyphenyl)-7-morpholinoquinolin-4-amine,
   - *N*-(3-(trifluoromethyl)phenyl)-7-morpholinoquinolin-4-amine,
   - *N⁴,N⁷*-bis(4-methoxyphenyl)quinolin-4,7-diamine,
   - *N⁴,N⁷*-bis(3-methoxyphenyl)quinolin-4,7-diamine,
   - *N⁴*-(3,5-difluorophenyl)-*N⁷*-(4-methoxyphenyl)quinolin-4,7-diamine,
   - *N⁴*-(3,5-difluorophenyl)-*N*⁷-(3-chloro-4-hydroxyphenyl)quinolin-4,7-diamine,
   - *N⁴*-(*p*-tolyl)-*N⁷*-(3-methoxyphenyl)quinolin-4,7-diamine,
   - *N⁴*-(*p*-tolyl)-*N⁷*-(4-methoxyphenyl)quinolin-4,7-diamine,
   - *N⁴*-(3-(trifluoromethyl)phenyl)-*N⁷*-(4-methoxyphenyl)quinolin-4,7-diamine,
   - *N⁴*-(*o*-tolyl)-*N⁷*-(3-methoxyphenyl)quinolin-4,7-diamine,
   - *N⁴*-(4-methoxyphenyl)-*N⁷*-(3-methoxyphenyl)quinolin-4,7-diamine,
   - *N⁴*-(3-methoxyphenyl)-*N⁷*-(4-methoxyphenyl)quinolin-4,7-diamine,
   - *N⁴*-(3,5-difluorophenyl)-*N⁷*-(3-methoxyphenyl)quinolin-4,7-diamine,
   - *N*-(4-methoxyphenyl)-7-chloro-2-methoxy-quinolin-4-amine,
   -*N*-(3,4-dimethoxyphenyl)-7-chloro-2-morpholino-quinolin-4-amine,
   - *N*-(3,4-dimethoxyphenyl)-7-chloro-2-(4-methylpiperazin-1-yl)-quinolin-4-amine,
   - *N*-(4-methoxyphenyl)-7-chloro-2-morpholino-quinolin-4-amine,
   - *N*-(4-methoxyphenyl)-7-chloro-2-(4-methylpiperazin-1-yl)-quinolin-4-amine,
   - *N*-(3,4-dimethoxyphenyl)-7-chloro-2-(2-morpholinoethoxy)quinolin-4-amine,
   - *N*-(3,4-dimethoxyphenyl)-7-chloro-2-methoxy-quinolin-4-amine,
   - 7-chloro-*N*-(2,3-dihydrobenzo[b][1,4]dioxin-6-yl)quinolin-4-amine, and
   - 7-chloro-*N*-(benzo[d][1,3]dioxol-5-yl)quinolin-4-amine;
   or a pharmaceutically acceptable salt and/or optical isomer, tautomer, solvate or isotopic variation thereof.
In aspect [12], the invention provides a pharmaceutical composition comprising a compound according to any one of the above aspects [1] to [8] and a pharmaceutically acceptable carrier.
In aspect [13], the invention provides the pharmaceutical composition according to aspect [12], additionally comprising another anti-cancer drug.
In aspect [14], the invention provides the pharmaceutical composition for use according to aspect [10] or [11], wherein said cancer is selected from melanoma, bladder, kidney, prostate, colon, lung, breast and blood cancer.
In aspect [15], the invention provides a compound of general formula (I): 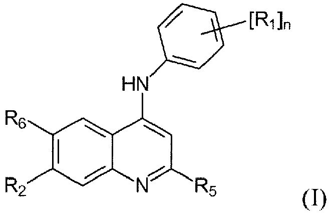 wherein
   R₁ is selected from H, Halo, OH, O-alkyl, NH₂, NH-alkyl, N-(alkyl)₂, S-alkyl, OCF₃, OCF₂H and O(CH₂)ₙ₁O(CH₂)ₙ₂CH₃, n₁ being 1 to 4 and n₂ being 0 to 3, Halo being selected from Cl, F, Br and I;
   n is 1 or 2,
   when n is 2, then it is also possible for R₁ to form with the phenyl group at the meta and para positions a dioxolane group or a 1,4 dioxane group,
   R₂ is selected from Halo and NR₃R₄, Halo being defined as previously,
   R₃ is H and R₄ is phenyl optionally substituted with one or more substituents selected from OH, O-alkyl and Halo,
   or R₃ and R₄ together form a (CH₂)₂O(CH₂)₂ chain, that is to say that NR₃R₄ forms a morpholino group 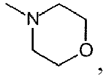
   R₅ is selected from H, cyclic radical, O-alkyl, O-(CH₂)ₙ₁-(cyclic radical), (CH₂)ₙ₁-(cyclic radical), n₁ being defined as previously, said cyclic radical being chosen from 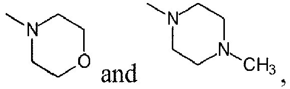
   R₆ is selected from H and Halo,
   with the proviso that when R₂ is F and R₅ = R₆ = H then R₁ is not 3-C1; 4-CI; 3-F; 4-OCH₃; 3-Cl and 4-F; 3-C1 and 4-Cl; or 4-F;
   and with the proviso that when R₂ is Cl and R₅ = R₆ = H then R₁ is not 4-OH or 4-Cl;
   or a pharmaceutically acceptable salt and/or optical isomer, tautomer, solvate or isotopic variation thereof,
   for use in the treatment of cancer.
In aspect [16], the invention provides the compound for use according to aspect [15], wherein said cancer is selected from melanoma, bladder, kidney, prostate, colon, lung, breast and blood cancer, preferably wherein said cancer is selected from melanoma and colon cancer.
In aspect [17], the invention provides the compound for use according to aspect [15] or [16], wherein said compound is selected from:
   - *N*-(3,5-difluorophenyl)-7-chloroquinolin-4-amine,
   - *N*-(4-methoxyphenyl)-7-chloroquinolin-4-amine,
   - *N*-(3-methoxyphenyl)-7-chloroquinolin-4-amine,
   - *N*-(3,5-difluorophenyl)-7-morpholinoquinolin-4-amine,
   - *N-*(4-(difluoromethoxy)phenyl)-7-chloro-quinolin-4-amine,
   - *N*-(4-methoxy-3-(2-methoxyethoxy)phenyl)-7-chloro-quinolin-4-amine,
   - *N*-(3,4-dimethoxyphenyl)-7-chloroquinolin-4-amine,
   - *N*-(2,4-dimethoxyphenyl)-7-chloroquinolin-4-amine,
   - *N*-(4-methoxyphenyl)-7-morpholinoquinolin-4-amine,
   - *N*-(3-methoxyphenyl)-7-morpholinoquinolin-4-amine,
   - *N⁴, N*⁷-bis(4-methoxyphenyl)quinolin-4,7-diamine,
   - *N⁴, N⁷*-bis(3-methoxyphenyl)quinolin-4,7-diamine,
   - *N⁴*-(3,5-difluorophenyl)-*N⁷*-(4-methoxyphenyl)quinolin-4,7-diamine,
   - *N⁴*-(3,5-difluorophenyl)-*N⁷*-(3-chloro-4-hydroxyphenyl)quinolin-4,7-diamine,
   - *N⁴*-(4-methoxyphenyl)-*N⁷*-(3-methoxyphenyl)quinolin-4,7-diamine,
   - *N⁴*-(3-methoxyphenyl)-*N⁷*-(4-methoxyphenyl)quinolin-4,7-diamine,
   - *N⁴*-(3,5-difluorophenyl)-*N⁷*-(3-methoxyphenyl)quinolin-4,7-diamine,
   - *N-*(4-methoxyphenyl)-7-chloro-2-methoxy-quinolin-4-amine,
   - *N*-(3,4-dimethoxyphenyl)-7-chloro-2-morpholino-quinolin-4-amine,
   - *N-*(3,4-dimethoxyphenyl)-7-chloro-2-(4-methylpiperazin-1-yl)-quinolin-4-amine,
   - *N*-(4-methoxyphenyl)-7-chloro-2-morpholino-quinolin-4-amine,
   - *N*-(4-methoxyphenyl)-7-chloro-2-(4-methylpiperazin-1-yl)-quinolin-4-amine,
   - *N*-(3,4-dimethoxyphenyl)-7-chloro-2-(2-morpholinoethoxy)quinolin-4-amine,
   - *N*-(3,4-dimethoxyphenyl)-7-chloro-2-methoxy-quinolin-4-amine,
   - 7-chloro-*N*-(2,3-dihydrobenzo[*b*][1,4]dioxin-6-yl)quinolin-4-amine, and
   - 7-chloro-*N-*(benzo[d][1,3]dioxol-5-yl)quinolin-4-amine;
   or a pharmaceutically acceptable salt and/or optical isomer, tautomer, solvate or isotopic variation thereof.
In aspect [18], the invention provides the compound for use according to any one of the above aspects [15] to [17], wherein said compound is to be administered in combination with an antibody selected from an anti-PD1 antibody, an anti-CTLA4 antibody, an anti-PD-L1 antibody and a mixture of two or more thereof.

### DETAILED DESCRIPTION OF THE INVENTION:

The invention relates to compounds of general formula (I): 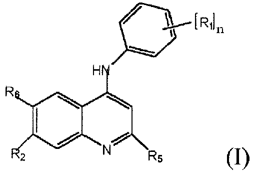 wherein
R₁ is selected from H, alkyl, Halo, OH, O-alkyl, NH₂, NH-alkyl, N-(alkyl)₂, S-alkyl, CF₃, OCF₃, OCF₂H and O(CH₂)ₙ₁O(CH₂)ₙ₂CH₃, n₁ being 1 to 4 and n₂ being 0 to 3, Halo being selected from Cl, F, Br and I;
n is 1 or 2,
when n is 2, then it is also possible for R₁ to form with the phenyl group at the meta and para positions a dioxolane group or a 1,4 dioxane group,
R₂ is selected from Halo and NR₃R₄, Halo being defined as previously,
R₃ is H and R₄ is phenyl optionally substituted with one or more substituents selected from OH, O-alkyl and Halo, or R₃ and R₄ together form a (CH₂)₂O(CH₂)₂ chain, that is to say that NR₃R₄ forms a morpholino group 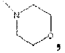
R₅ is selected from H, cyclic radical, O-alkyl, O-(CH₂)ₙ₁-(cyclic radical), (CH₂)ₙ₁-(cyclic radical), n₁ being defined as previously, said cyclic radical being chosen from 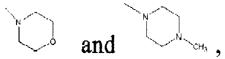
R₆ is selected from H and Halo,
with the proviso that when R₂ is Halo or R₆ is Halo then R₅ is not H,
its pharmaceutically acceptable salts and/or optical isomers, tautomers, solvates or isotopic variations thereof.

In the above general formula (I), unless specified otherwise:
Alkyl denotes a straight chain or branched group containing 1, 2, 3, 4, 5 or 6 carbon atoms. Examples of suitable alkyl radicals are methyl, ethyl, n-propyl, cyclopropyl, isopropyl, n-butyl, isobutyl, sec-butyl, tert-butyl, n-pentyl, cyclopentyl, isopentyl, n-hexyl, cyclohexyl, etc.

Alkyl is preferably a straight chain group comprising from 1 to 4 carbon atoms. Alkyl is preferably methyl.

The free bond on the phenyl group means that the phenyl may be substituted in the ortho, meta or para position and when n is 2, in two of the ortho, meta or para positions.

Halo means fluoro, chloro, bromo and iodo. A preferred Halo group for R₁ is fluoro or chloro, and more preferably chloro.

A preferred halo group for R₂ is chloro.

A first preferred group of compounds includes compounds of general formula (2): 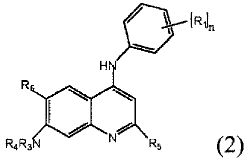 wherein R₁, R₅, R₆ and n are as defined above, R₂ is NR₃R₄ and R₃, R₄ are as defined above. A second preferred group of compounds includes compounds of general formula (3) 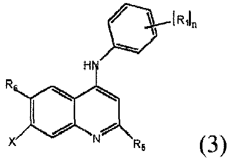 wherein R₁, R₅, R₆ and n are as defined above, R₂ is X, X is Halo as defined above, and R₅ is not H. Preferably X is chloro.

According to an embodiment of the invention, R₁ is selected from methyl (CH₃), fluoro (F), chloro (Cl), hydroxy (OH), methoxy (OCH₃), CF₃, OCF₃, OCF₂H and O(CH₂)₂OCH₃, and when n is 2 then the substituents R₁ are identical or different.

According to another embodiment of the invention, when n is 2 then the two R₁ form with the phenyl group at the meta and para positions a dioxolane group or a 1,4 dioxane group.

According to an embodiment of the invention, in the compounds defined above, when R₂ is NR₃R₄, then R₃ is H and R₄ is a phenyl unsubstituted or substituted with one or two groups, identical or different, selected from OH, OCH₃ and Halo, Halo being preferably chloro.

According to another embodiment of the invention, in the compounds defined above, when R₂ is NR₃R₄, then R₃ and R₄ together form a (CH₂)₂O(CH₂)₂ chain, that is to say that NR₃R₄ forms a morpholino group

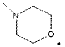

According to an advantageous embodiment of the invention, preferred compounds are those of general formula (2), wherein R₂ is NR₃R₄ and forms a morpholino group, said compounds being more particularly the following:
- *N*-(3,5-difluorophenyl)-7-morpholinoquinolin-4-amine (compound 10),
- *N-*(*p*-tolyl) 7-morpholinoquinolin-4-amine (compound 11),
- *N*-(*o*-tolyl)-7-morpholinoquinolin-4-amine (compound 12),
- *N*-(4-methoxyphenyl)-7-morpholinoquinolin-4-amine (compound 13),
- *N*-(3-methoxyphenyl)-7-morpholinoquinolin-4-amine (compound 14) and
- *N*-(3-(trifluoromethyl)phenyl)-7-morpholinoquinolin-4-amine (compound 15).

According to another advantageous embodiment of the invention, preferred compounds are those of general formula (2), wherein R₂ is NR₃R₄, R₃ being H and R₄ a phenyl unsubstituted or substituted with one or two groups as defined above, said compounds being more particularly the following:
- *N*⁴*, N*⁷-bis(4-methoxyphenyl)quinolin-4,7-diamine (compound 16),
- *N*⁴, *N*⁷-bis(3-methoxyphenyl)quinolin-4,7-diamine (compound 17),
- *N*⁴-(3,5-difluorophenyl)-*N*⁷-(4-methoxyphenyt)quinolin-4,7-diamine (compound 18),
- *N*⁴-(3,5-difluorophenyl)-*N*⁷-(3-chloro-4-hydroxyphcnyl)quinolin-4,7-diaminc (compound 19) (also named 2-chloro-4-((4-((3,5-difluorophenyl)amino)quinolin-7-yl)amino)phenol),
- *N*⁴-(*p*-tolyl)-*N*⁷-(3-methoxyphenyl)quinolin-4,7-diamine (compound 20),
- *N*⁴-(*p*-tolyl)-*N*⁷-(4-methoxyphenyl)quinolin-4,7-diamine (compound 21),
- *N*⁴-(3-(trifluoromethyl)phenyl)-*N*⁷-(4-methoxyphenyl)quinolin-4,7-diamine (compound 22),
- *N*⁴-(*o*-tolyl)-*N*⁷-(3-methoxyphenyl)quinolin-4,7-diamine (compound 23),
- *N*⁴-(4-methoxyphenyl)-*N*⁷-(3-methoxyphenyl)quinolin-4,7-diamine (compound 24),
- *N*⁴-(3-methoxyphenyl)-*N*⁷-(4-methoxyphenyl)quinolin-4,7-diamine (compound 25) and
- *N⁴*-(3,5-difluorophenyl)-*N⁷*-(3-methoxyphenyl)quinolin-4,7-diamine (26).

According to another advantageous embodiment of the invention, preferred compounds are those of general formula (3) which are more particularly the following:
- *N*-(4-methoxyphenyl)-7-chloro-2-methoxy-quinolin-4-amine (compound 27),
- *N*-(3,4-dimethoxyphenyl)-7-chloro-2-morpholino-quinolin-4-amine (compound 28),
- *N*-(3,4-dimethoxyphenyl)-7-chloro-2-(4-methylpiperazin-1-yl)-quinolin-4-amine (compound 29),
- *N*-(4-methoxyphenyl)-7-chloro-2-morpholino-quinolin-4-amine (compound 30),
- *N*-(4-methoxyphenyl)-7-chloro-2-(4-methylpiperazin-1-yl)-quinolin-4-amine (compound 31),
- *N*-(3,4-dimethoxyphenyl)-7-chloro-2-(2-morpholinoethoxy)quinolin-4-amine (compound 32) and
- *N*-(3,4-dimethoxyphenyl)-7-chloro-2-methoxy-quinolin-4-amine (compound 33).

The compounds of formula (I), and their pharmaceutically acceptable salts, are valuable pharmaceutically active compounds suitable for the therapy and prophylaxis of various cancers.

The invention thus also pertains to compounds of formula (I) as defined above and, if appropriate, their pharmaceutically acceptable salts and/or optical isomers, tautomers, solvates or isotopic variations thereof for use in the treatment of cancer, namely solid tumor cancer, and preferably those selected from melanoma, bladder, kidney, prostate, colon, lung, breast and blood cancer.

Compounds of the invention may be administered as crystalline or amorphous products. They may be obtained, for example, as solid plugs, powders, or films by methods such as precipitation, crystallization, freeze-drying, spray drying, or evaporative drying. Microwave or radio frequency drying may be used for this purpose.

They may be administered alone or in combination with one or more other compounds of the invention or in combination with one or more other drugs (or as any combination thereof). Generally, they will be administered as a formulation in association with one or more pharmaceutically acceptable excipients. The term "excipient" is used herein to describe any ingredient other than the compound(s) of the invention. The choice of excipient will to a large extent depend on factors such as the particular mode of administration, the effect of the excipient on solubility and stability, and the nature of the dosage form. Pharmaceutical compositions suitable for the delivery of compounds of the present invention and methods for their preparation will be readily apparent to those skilled in the art. Such compositions and methods for their preparation may be found, for example, in 'Remington's Pharmaceutical Sciences', 19th Edition (Mack Publishing Company, 1995).

Another aspect of the invention is thus a pharmaceutical composition comprising a compound of general formula (1) as defined above and a pharmaceutically acceptable carrier.

The compounds of the invention may be administered by any suitable route.

Thus, a compound of the invention may be formulated as a pharmaceutical composition for oral, buccal, intranasal, parenteral (e. g. intravenous, intramuscular or subcutaneous), topical or rectal administration or in a form suitable for administration by inhalation or insufflation.

For oral administration, the pharmaceutical composition may take the form of, for example, a tablet or capsule prepared by conventional means with a pharmaceutically acceptable excipient such as a binding agent (e. g., pregelatinized maize starch, polyvinylpyrrolidone or hydroxypropyl methylcellulose); filler (e. g., lactose, microcrystalline cellulose or calcium phosphate); lubricant (e. g., magnesium stearate, talc or silica); disintegrant (e. g., potato starch or sodium starch glycolate); or wetting agent (e. g., sodium lauryl sulphate).

The tablets may be coated by methods well known in the art. Liquid preparations for oral administration may take the form of a, for example, solution, syrup or suspension, or they may be presented as a dry product for constitution with water or other suitable vehicle before use. Such liquid preparations may be prepared by conventional means with a pharmaceutically acceptable additive such as a suspending agent (e. g., sorbitol syrup, methyl cellulose or hydrogenated edible fats); emulsifying agent (e. g., lecithin or acacia); non-aqueous vehicle (e. g., almond oil, oily esters or ethyl alcohol); and preservative (e. g., methyl or propyl p-hydroxybenzoates or sorbic acid).

For buccal administration, the composition may take the form of tablets or lozenges formulated in conventional manner. A compound of the present invention may also be formulated for sustained delivery according to methods well known to those of ordinary skill in the art. Examples of such formulations can be found in United States Patents 3,538, 214, 4,060, 598,4, 173,626, 3,119, 742, and 3,492,397.

A compound of the invention may be formulated for parenteral administration by injection, including using conventional catheterization techniques or infusion. Formulations for injection may be presented in unit dosage form, e.g., in ampules or in multi-dose containers, with an added preservative. The compositions may take such forms as suspensions, solutions or emulsions in oily or aqueous vehicles, and may contain a formulating agent such as a suspending, stabilizing and/or dispersing agent. Alternatively, the active ingredient may be in powder form for reconstitution with a suitable vehicle, e. g., sterile pyrogen-free water, before use parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water.

Inasmuch as it may desirable to administer a combination of active compounds, for example, for the purpose of treating a particular disease or condition, it is within the scope of the present invention that two or more pharmaceutical compositions, at least one of which contains a compound in accordance with the invention, may conveniently be combined in the form of a kit suitable for coadministration of the compositions. Thus, the kit of the invention comprises two or more separate pharmaceutical compositions, at least one of which contains a compound of formula (1) in accordance with the invention, and means for separately retaining said compositions, such as a container, divided bottle, or divided foil packet. An example of such a kit is the familiar blister pack used for the packaging of tablets, capsules and the like.

The kit of the invention is particularly suitable for administering different dosage forms, for example parenteral, for administering the separate compositions at different dosage intervals, or for titrating the separate compositions against one another. To assist compliance, the kit typically comprises directions for administration and may be provided with a so-called memory aid.

For administration to human patients, the total daily dose of the compounds of the invention is typically in the range 0.001 mg to 5000 mg depending, of course, on the mode of administration. For example, an intravenous daily dose may only require from 0.001 mg to 40 mg. The total daily dose may be administered in single or divided doses and may, at the physician's discretion, fall outside of the typical range given herein.

These dosages are based on an average human subject having a weight of about 65 kg to 70 kg. The physician will readily be able to determine doses for subjects whose weight falls outside this range, such as infants and the elderly.

For the avoidance of doubt, references herein to "treatment" include references to curative, palliative and prophylactic treatment.

According to another embodiment of the present invention, the compounds of the formula (I), or pharmaceutically acceptable salts or compositions thereof, can also be used as a combination with one or more additional therapeutic agents to be co-administered to a patient to obtain some particularly desired therapeutic end result such as the treatment of cancers, including solid tumor cancers, such as melanoma, bladder, kidney, prostate, colon, pancreas, lung and blood cancers, such as Hodgkin disease.

Preferably, the compounds of the invention either alone or in combination are administered to patients at metastatic stage suffering from melanoma, renal cancer, colorectal cancer, lung cancer, specifically non-small lung cancer and prostate cancer.

The second and more additional therapeutic agents may also be compounds of the formula (1), or a pharmaceutically acceptable salt thereof, or one or more compounds known in the art for the treatment of the conditions listed above. More typically, the second and more therapeutic agents will be selected from a different class of therapeutic agents.

As used herein, the terms "co-administration", "co-administcrcd" and "in combination with", referring to the compounds of formula (I) and one or more other therapeutic agents, is intended to mean, and does refer to and include the following: simultaneous administration of such combination of compound(s) of formula (1) and therapeutic agent(s) to a patient in need of treatment, when such components are formulated together into a single dosage form which releases said components at substantially the same time to said patient, substantially simultaneous administration of such combination of compound(s) of formula (I) and therapeutic agent(s) to a patient in need of treatment, when such components are formulated apart from each other into separate dosage forms which are taken at substantially the same time by said patient, whereupon said components are released at substantially the same time to said patient, sequential administration of such combination compound(s) of formula (I) and therapeutic agent(s) to a patient in need of treatment, when such components are formulated apart from each other into separate dosage forms which are taken at consecutive times by said patient with a significant time interval between each administration, whereupon said components are released at substantially different times to said patient; and sequential administration of such combination of compound(s) of formula (I) and therapeutic agent(s) to a patient in need of treatment, when such components are formulated together into a single dosage form which releases said components in a controlled manner whereupon they are concurrently, consecutively, and/or administered at the same and/or different times by said patient, where each part may be administered by either the same or different route.

Suitable examples of other therapeutic agents which may be used in combination with the compound(s) of formula (1), or pharmaceutically acceptable salts or compositions thereof, include, but are by no means limited to:
- Anti-cancer agents used for the therapy of cancers such as dacarbazine,
- Nitrosourea alkylating agents, such as fotemustine,
- BRAF inhibitors such as vemurafenib or dabrafenib,
- MEK inhibitors such as trametinib,
- Anti- CTLA4 antibodies, such as ipilimumab,
- Anti PD1 antibodies, such as nivolumab, pidizilumab, pembrolizumab and AMP-514,
- Anti PD1 fusion protein such as AMP-224,
- Anti PD-L1 antibodies, such as atezolizumab, durvalumab, avelumab, utomilumab and MPDL3280A,
- Other immune checkpoint blocking agents or, in general, therapeutic agents based on immune approaches for treating cancer.

The compounds of the invention are co-administered with an antibody selected from an anti-PD1antibody, an anti-CTLA4 antibody and an anti-PD-L1 antibody and a mixture of two or more thereof.

According to a specific embodiment, the present invention relates to pharmaceutical composition as a combined preparation for simultaneous, separate or sequential use in the treatment of cancer, comprising:
- a compound of general formula (I) 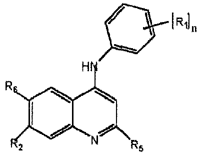 wherein
   R₁ is selected from H, alkyl, Halo, OH, O-alkyl, NH₂, NH-alkyl, N-(alkyl)₂, S-alkyl, CF₃, OCF₃, OCF₂H and O(CH₂)ₙ₁O(CH₂)ₙ₂CH₃, n₁ being 1 to 4 and n₂ being 0 to 3, Halo being selected from Cl, F, Br and I;
   n is 1 or 2;
   when n is 2, then it is also possible for R₁ to form with the phenyl group at the meta and para positions a dioxolane group or a 1,4 dioxane group,
   R₂ is selected from H, Halo and NR₃R₄; Halo being defined as previously,
   R₃ is H and R₄ is phenyl optionally substituted with one or more substituents selected from OH, O-alkyl and Halo, or R₃ and R₄ together form a (CH₂)₂O(CH₂)₂ chain,
   R₅ is selected from H, cyclic radical, O-alkyl, O-(CH₂)ₙ₁-(cyclic radical), (CH₂)ₙ₁-(cyclic radical), n₁ being defined as previously, said cyclic radical being chosen from 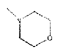 and 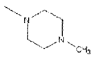 ,
   R₆ is selected from H and Halo,
      or a pharmaceutically acceptable salt and/or optical isomer, tautomer, solvate or isotopic variation thereof,
- at least one antibody selected from an anti-PD1 antibody, an anti-CTLA4 antibody, an anti-PD-L1 antibody and a mixture of two or more thereof, and
- optionally at least one pharmaceutically acceptable carrier.

According to an advantageous embodiment of the invention, preferred compounds used in the above mentioned pharmaceutical composition are those of general formula (1) which are more particularly the following:
- *N*-(3,5-difluorophenyl)-7-chloroquinolin-4-amine (compound 1),
- *N*-(4-methylphenyl)-7-chloroquinolin-4-amine (compound 2),
- *N*-(3-trifluoromethylphenyl)-7-chloroquinotin-4-amine (compound 3),
- *N*-(2-methylphenyl)-7-chloroquinolin-4-amine (compound 4),
- *N*-(4-methoxyphenyl)-7-chloroquinolin-4-amine (compound 5),
- *N*-(3-methoxyphenyl)-7-chloroquinolin-4-amine (compound 6),
- *N*-(4-hydroxyphenyl)-7-chloroquinolin-4-amine (compound 7),
- *N*-(4-hydroxy-3-chlorophenyl)-7-chloroquinolin-4-amine (compound 9),
- *N-*(4-trifluoromethylphenyl)-7-chloroquinolin-4-amine (compound 9),
- *N*-(3,5-difluorophenyl)-7-morpholinoquinolin-4-amine (compound 10),
- *N*-(4-(difluoromethoxy)phenyl)-7-chloro-quinolin-4-amine (compound 34),
- *N*-(4-methoxy-3-(2-methoxyethoxy)phenyl)-7-chloro-quinolin-4-amine (compound 35),
- *N*-(4-trifluoromethoxyphenyl)-6-chloroquinolin-4-amine (compound 36),
- *N*-(2,4-dimethoxyphenyl)-6-chloroquinolin-4-amine (compound 37),
- *N*-(3,4-dimethoxyphenyl)-7-chloroquinolin-4-amine (compound 38),
- *N*-(2,4-dimethoxyphenyl)-7-chloroquinolin-4-amine (compound 39),
- *N*-(*p*-tolyl) 7-morpholinoquinolin-4-amine (compound 11),
- *N-*(*o*-tolyl)-7-morpholinoquinolin-4-amine (compound 12),
- *N*-(4-methoxyphenyl)-7-morpholinoquinolin-4-amine (compound 13),
- *N*-(3-methoxyphenyl)-7-morpholinoquinolin-4-amine (compound 14),
- *N*-(3-(trifluoromethyl)phenyl)-7-morpholinoquinolin-4-amine (compound 15),
*- N*⁴, *N*⁷-bis(4-methoxyphenyl)quinolin-4,7-diamine (compound 16),
- *N*⁴*, N*⁷-bis(3-methoxyphenyl)quinolin-4,7-diamine (compound 17),
- *N*⁴-(3,5-difluorophenyl)-*N*⁷-(4-methoxyphenyl)quinolin-4,7-diamine (compound 18),
- *N⁴*-(3,5-difluorophenyl)-*N⁷*-(3-chloro-4-hydroxyphenyl)quinolin-4,7-diamine (compound 19),
- *N*⁴-(*p*-tolyl)-*N*⁷-(3-methoxyphenyl)quinolin-4,7-diamine (compound 20),
- *N*⁴-(*p*-tolyl)-*N*⁷-(4-methoxyphenyl)nuinolin-4,7-diamine (compound 21),
- *N*⁴-(3-(trifluoromethyl)phenyl)-*N*⁷-(4-methoxyphenyl)quinolin-4,7-diamine (compound 22),
- *N*⁴-(*o*-tolyl)-*N*⁷-(3-methoxyphenyl)quinolin-4,7-diamine (compound 23),
- *N*⁴-(4-methoxyphenyl)-*N*⁷-(3-methoxyphcnyl)quinolin-4,7-diamine (compound 24),
- *N*⁴-(3-methoxyphenyl)-*N*⁷-(4-methoxyphenyl)quinolin-4,7-diamine (compound 25),
- *N*⁴-(3,5-difluorophenyl)-*N*⁷-(3-methoxyphenyl)quinolin-4,7-diamine (compound 26),
- *N*-(4-methoxyphenyl)-7-chloro-2-methoxy-quinolin-4-amine (compound 27),
- *N*-(3,4-dimethoxyphenyl)-7-chloro-2-morpholino-quinolin-4-amine (compound 28),
- *N*-(3,4-dimethoxyphenyl)-7-chloro-2-(4-methylpiperazin-1-yl)-quinolin-4-amine (compound 29),
- *N*-(4-methoxyphenyl)-7-chloro-2-morpholino-quinolin-4-amine (compound 30),
- *N*-(4-methoxyphenyl)-7-chloro-2-(4-methylpiperazin-1-yl)-quinolin-4-amine (compound 31),
- *N*-(3,4-dimethoxyphenyl)-7-chloro-2-(2-morpholinoethoxy)quinolin-4-amine (compound 32),
- *N*-(3,4-dimethoxyphenyl)-7-chloro-2-methoxy-quinolin-4-amine (compound 33),
- 7-chloro-*N*-(2,3-dihydrobenzo[b][1,4]dioxin-6-yl)quinolin-4-amine (40),
- 7-chloro-*N*-(benzo[d][1,3]dioxol-5-yl)quinolin-4-amine (41).

The last two compounds (40) and (41) correspond respectively to compounds (1) as defined above wherein, when n is 2, then R₁ form respectively with the phenyl group at the meta and para positions a 1,4 dioxane group (compound 40) or a dioxolane group (compound 41).

The present invention also relates to a pharmaceutical composition as defined above which additionally comprising another anti-cancer drug.

The invention more particularly relates to a pharmaceutical composition as defined above, as a combined preparation for simultaneous, separate or sequential use in the treatment of cancer, said being preferably selected from melanoma, bladder, kidney, prostate, colon, lung, breast and blood cancer.

According to another specific embodiment, the invention relates to a compound of general formula (I): 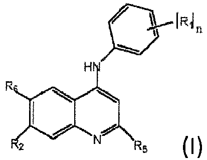 wherein
R₁ is selected from H, Halo, OH, O-alkyl, NH₂, NH-alkyl, N-(alkyl)₂, S-alkyl, OCF₃, OCF₂H and O(CH₂)ₙ₁O(CH₂)ₙ₂CH₃, n₁ being 1 to 4 and n₂ being 0 to 3, Halo being selected from Cl, F, Br and I;
n is 1 or 2,
when n is 2, then it is also possible for R₁ to form with the phenyl group at the meta and para positions a dioxolane group or a 1,4 dioxane group,
R₂ is selected from Halo and NR₃R₄, Halo being defined as previously,
R₃ is H and R₄ is phenyl optionally substituted with one or more substituents selected from OH, O-alkyl and Halo,
or R₃ and R₄ together form a (CH₂)₂O(CH₂)₂ chain, that is to say that NR₃R₄ forms a morpholino group 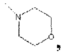
R₅ is selected from H, cyclic radical, O-alkyl, O-(CH₂)ₙ₁-(cyclic radical), (CH₂)ₙ₁-(cyclic radical), n₁ being defined as previously, said cyclic radical being chosen from 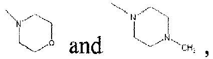
R₆ is selected from H and Halo,
with the proviso that when R₂ is F and R₅ = R₆ = H then R₁ is not 3-Cl ; 4-Cl ; 3-F ; 4-OCH₃ ; 3-Cl and 4-F ; 3-Cl and 4-Cl ; or 4-F ;
and with the proviso that when R₂ is Cl and R₅ = R₆ = H then R₁ is not 4-OH or 4-Cl ;
   or a pharmaceutically acceptable salt and/or optical isomer, tautomer, solvate or isotopic variation thereof,
for use in the treatment of cancer, said cancer being preferably selected from melanoma, bladder, kidney, prostate, colon, lung, breast and blood cancer, and more preferably selected from melanoma and colon cancer.

More particularly, the compounds for use as defined above are chosen from the following compounds:
- *N*-(3,5-difluorophenyl)-7-chloroquinolin-4-amine,
- *N*-(4-methoxyphenyl)-7-chloroquinolin-4-amine,
- *N*-(3-methoxyphenyl)-7-chloroquinolin-4-amine,
- *N*-(3,5-difluorophenyl)-7-morpholinoquinolin-4-amine,
- *N*-(4-(difluoromethoxy)phenyl)-7-chloro-quinolin-4-amine,
- *N*-(4-methoxy-3-(2-methoxyethoxy)phenyl)-7-chloro-quinolin-4-amine,
- *N*-(3,4-dimethoxyphenyl)-7-chloroquinolin-4-amine,
- *N*-(2,4-dimethoxyphenyl)-7-chloroquinolin-4-amine,
- *N*-(4-methoxyphenyl)-7-morpholinoquinolin-4-amine,
- *N*-(3-methoxyphenyl)-7-morpholinoquinolin-4-amine,
- *N⁴, N*⁷-bis(4-methoxyphenyl)quinolin-4,7-diamine,
- *N⁴, N⁷*-bis(3-methoxyphenyl)quinolin-4,7-diamine,
- *N⁴*-(3,5-difluorophenyl)-*N⁷*-(4-methoxyphenyl)quinolin-4,7-diamine,
- *N*⁴-(3,5-difluorophenyl)-*N⁷*-(3-chloro-4-hydroxyphenyl)quinolin-4,7-diamine,
- *N⁴*-(4-methoxyphenyl)-*N⁷*-(3-methoxyphenyl)quinolin-4,7-diamine,
- *N⁴*-(3-methoxyphenyl)-*N⁷*-(4-methoxyphenyl)quinolin-4,7-diamine,
- *N⁴*-(3,5-difluorophenyl)-*N⁷*-(3-methoxyphenyl)quinolin-4,7-diamine,
- *N*-(4-methoxyphenyl)-7-chloro-2-methoxy-quinolin-4-amine,
- *N*-(3,4-dimethoxyphenyl)-7-chloro-2-morpholino-quinolin-4-amine,
- *N*-(3,4-dimethoxyphenyl)-7-chloro-2-(4-methylpiperazin-1-yl)-quinolin-4-amine,
- *N*-(4-methoxyphenyl)-7-chloro-2-morpholino-quinolin-4-amine,
- *N*-(4-methoxyphenyl)-7-chloro-2-(4-methylpiperazin-1-yl)-quinolin-4-amine,
- *N*-(3,4-dimethoxyphenyl)-7-chloro-2-(2-morpholinoethoxy)quinolin-4-amine,
- *N*-(3,4-dimethoxyphenyl)-7-chloro-2-methoxy-quinolin-4-amine,
- 7-chloro-*N*-(2,3-dihydrobenzo[*b*][1,4]dioxin-6-yl)quinolin-4-amine, and
- 7-chloro-*N*-(benzo[d][1,3]dioxol-5-yl)quinolin-4-amine.

It is to be appreciated that all references herein to treatment include curative, palliative and prophylactic treatment. The description, which follows, concerns the therapeutic applications to which the compounds of formula (1) may be put.

A still further aspect of the present invention also relates to the compounds of formula (I), or pharmaceutically acceptable salts or compositions thereof, for use as a drug having an anticancer activity, particularly for use in
the treatment of a solid tumor cancer selected melanoma, colon, lung and breast cancer.

The compounds of the formula (1) may be prepared using conventional procedures such as by the following illustrative methods in which the various substituents are as previously defined for the compounds of the formula (I) unless otherwise stated.

The procedure for preparing the compounds of the invention, wherein R₅ = R₆ = H and R₂ is chloro or NR₃R₄ comprises the following steps:
i) Reacting 4,7-dichloroquinoline with a compound of formula 4: 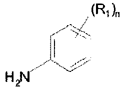 to obtain a compound of formula 3: 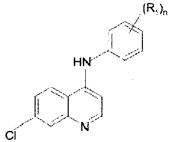
ii) If appropriate, reacting the compound of formula 3 with a compound of general formula 5: HNR₃R₄
iii) Obtaining the compound of formula (2), that is subsequently isolated and purified.

Step i) is performed in the conditions of an aromatic nucleophilic substitution in an acidic medium (Journal of the American Chemical Society, 1946, vol 68, 1807-1808).

Step ii) is performed in the conditions of a Buchwald-Hartwig coupling (Angewandte Chemie, International edition,1995, vol 34,1348-1350) in the presence of a catalyst, such as palladium and a base in a suitable solvent, e.g. Toluene, DME or dioxane.

Compounds of general formula 3 wherein R₅ = R₆ = H and X is fluoro or bromo, can be prepared starting from the corresponding 4-chloro-7-haloquinoline, according to the previously reported procedures (see for examples: Bioorg. Med. Chem. 2013, 21 (11), 3147-3153 ; J. Med. Chem., 2015, 58 (14), 5522-5537).

Compounds of general formula (I) wherein R₂ = R₅ = H and R₂ is Halo can be prepared starting from the corresponding 4-chloro-6-haloquinoline, according to the procedure described above for compound of formula (3).

Compounds of general formula (1) wherein R₅ ≠ H, R₂ = Cl and R₆ = H can be prepared by the following steps:
i) Reacting 2,4,7-trichloroquinoline with a compound of formula 4: 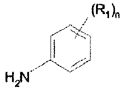 to obtain a compound of formula 6 : 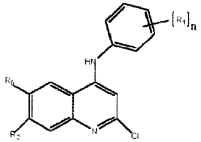
ii) If appropriate, reacting the compound of formula 6 with a compound of general formula : HNR₃R₄ or O-alkyl.

Step i) is performed in the conditions of an aromatic substitution in basic medium in DMA (Tetrahedron Letters, 2013, vol 54, 6900-6904).

Step ii) is performed in the conditions of an aromatic substitution.

Pharmaceutically acceptable salts of the compounds of formula (I) include the acid addition and base salts thereof.

Suitable acid addition salts are formed from acids, which form non-toxic salts.

Examples include the acetate, aspartate, benzoate, besylate, bicarbonate/carbonate, bisulphate/sulphate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, saccharate, stearate, succinate, tartrate, tosylate and trifluoroacetate and xinafoate salts.

Suitable base salts are formed from bases, which form non-toxic salts.

Examples include the aluminium, arginine, benzathine, calcium, choline, diethylamine, diolamine, glycine, lysine, magnesium, meglumine, olamine, potassium, sodium, tromethamine and zinc salts. Hemisalts of acids and bases may also be formed, for example, hemisulphate and hemicalcium salts.

For a review on suitable salts, see "Handbook of Pharmaceutical Salts: Properties, Selection, and Use" by Stahl and Wermuth (Wiley-VCH, Weinheim, Germany, 2002).

Pharmaceutically acceptable salts of compounds of formula (I) may be prepared by one or more of three methods:
(i) by reacting the compound of formula (I) with the desired acid or base;
(ii) by removing an acid- or base-labile protecting group from a suitable precursor of the compound of formula (I) or by ring-opening a suitable cyclic precursor, for example, a lactone or lactam, using the desired acid or base; or
(iii) by converting one salt of the compound of formula (I) to another by reaction with an appropriate acid or base or by means of a suitable ion exchange column.

All three reactions are typically carried out in solution. The resulting salt may precipitate out and be collected by filtration or may be recovered by evaporation of the solvent. The degree of ionization in the resulting salt may vary from completely ionized to almost non-ionized.

The compounds of the invention may exist in both unsolvated and solvated forms.

The term 'solvate' is used herein to describe a molecular complex comprising the compound of the invention and a stoichiometric amount of one or more pharmaceutically acceptable solvent molecules, for example, ethanol. The term 'hydrate' is employed when said solvent is water.

Included within the scope of the invention are complexes such as clathrates, drug-host inclusion complexes wherein, in contrast to the aforementioned solvates, the drug and host are present in stoichiometric or non-stoichiometric amounts. Also included are complexes of the drug containing two or more organic and/or inorganic components, which may be in stoichiometric or non-stoichiometric amounts. The resulting complexes may be ionised, partially ionized, or non-ionized. For a review of such complexes, see J Pharm Sci, 64 (8), 1269-1288 by Haleblian (August 1975).

Hereinafter all references to compounds of formula (I) include references to salts, solvates and complexes thereof and to solvates and complexes of salts thereof.

The compounds of the invention include compounds of formula (1) as hereinbefore defined, including all polymorphs and crystal habits thereof, isomers thereof (including optical, geometric and tautomeric isomers) as hereinafter defined and isotopically-labeled compounds of formula (1).

Also disclosed herein are so-called 'pro-drugs' of the compounds of formula (I).

Further information on the use of prodrugs may be found in 'Pro-drugs as Novel Delivery Systems, Vol. 14, ACS Symposium Series (T. Higuchi and W. Stella) and 'Bioreversible Carriers in Drug Design', Pergamon Press, 1987 (ed. E. B Roche, American Pharmaceutical Association).

Prodrugs as disclosed herein can, for example, be produced by replacing appropriate functionalities present in the compounds of formula (1) with certain moieties known to those skilled in the art as 'pro-moieties' as described, for example, in "Design of Prodrugs" by H. Bundgaard (Elsevier, 1985).

Some examples of prodrugs disclosed herein include:
(i) where the compound of formula (1) contains a carboxylic acid functionality (COOH), an ester thereof, for example, a compound wherein the hydrogen of the carboxylic acid functionality of the compound of formula (I) is replaced by (C1-C8)alkyl;
(ii) where the compound of formula (1) contains an alcohol functionality (-OH), an ether thereof, for example, a compound wherein the hydrogen of the alcohol functionality of the compound of formula (1) is replaced by (C1-C6)alkanoyloxymethyl; and
(iii) where the compound of formula (I) contains a primary or secondary amino functionality, an amide thereof, for example, a compound wherein, as the case may be, one or both hydrogens of the amino functionality of the compound of formula (I) is/are replaced by (C₁-C₁₀)alkanoyl.

Further examples of replacement groups in accordance with the foregoing examples and examples of other prodrug types may be found in the aforementioned references. Moreover, certain compounds of formula (I) may themselves act as prodrugs of other compounds of formula (I).

Also disclosed herein are metabolites of compounds of formula (1), that is, compounds formed in vivo upon administration of the drug. Some examples of metabolites disclosed herein include:
(i) Where the compound of formula (I) contains a methyl group, an hydroxymethyl derivative thereof;
(ii) Where the compound of formula (I) contains a tertiary amino group, a secondary amino derivative thereof;
(iii) Where the compound of formula (I) contains a secondary amino group, a primary derivative thereof;
(iv) Where the compound of formula (1) contains a phenyl moiety, a phenol derivative thereof.

Compounds of formula (1) containing one or more asymmetric carbon atoms can exist as two or more stereoisomers. Included within the scope of the present invention are all stereoisomers, geometric isomers and tautomeric forms of the compounds of formula (I), including compounds exhibiting more than one type of isomerism, and mixtures of one or more thereof. Also included is acid addition or base salts wherein the counter ion is optically active, for example, d-lactate or l-lysine, or racemic, for example, dl-tartrate or dl-arginine. Conventional techniques for the preparation/isolation of individual enantiomers include chiral synthesis from a suitable optically pure precursor or resolution of the racemate (or the racemate of a salt or derivative) using, for example, chiral high-pressure liquid chromatography (HPLC).

Alternatively, the racemate (or a racemic precursor) may be reacted with a suitable optically active compound, for example, an alcohol, or, in the case where the compound of formula (1) contains an acidic or basic moiety, an acid or base such as tartaric acid or 1-phenylethylamine. The resulting diastereomeric mixture may be separated by chromatography and/or fractional crystallization and one or both of the diastereoisomers converted to the corresponding pure enantiomer(s) by means well known to a skilled person.

Chiral compounds of the invention (and chiral precursors thereof) may be obtained in enantiomerically-enriched form using chromatography, typically HPLC (chiral columns), on an asymmetric resin with a mobile phase consisting of a hydrocarbon, typically heptane or hexane, containing from 0 to 50% by volume of isopropanol, typically from 2% to 20%, and from 0 to 5% by volume of an alkylamine, typically 0.1% diethylamine. For reverse HPLC CH₃CN and H₂O, MeOH or iPrOH and H₂O are used as solvents. Concentration of the eluate affords the enriched mixture.

Stereoisomeric conglomerates may be separated by conventional techniques known to those skilled in the art-see, for example, "Stereochemistry of Organic Compounds" by E. L. Eliel (Wiley, New York, 1994). "Chiral Separation Techniques". by G. Subramanian. John Wiley & Sons, 2008. "Preparative Enantioselective Chromatography" by G. B. Cox. Wiley, 2005.

Pharmaceutically acceptable solvates in accordance with the invention include those wherein the solvent of crystallization may be isotopically substituted, e.g. D₂O.

The following examples illustrate the preparation of the compounds of the formula (1) and their pharmacological properties.

### FIGURES:

**Figure 1****. Compound 5 displays the same secretory phenotype and senescence-inducing capacity as the knockdown of the NF-kB non-canonical pathway in different cancer cells.**
   **A/B.** mRNA levels of EZH2 (**A**) or CDKN1A and IFNG (**B**) in A375 cells treated for 96 h with increasing concentrations of Compound 5. **C.** IFN-γ measured by ELISA in different cell lines treated with Compound 5 or DMSO for 96 h.
   * p<0.05 against respective controls.
**Figure 2****. Compound 5 potentiates the anti-PD1 immune response in Balb/c mice subcutaneously injected with CT26 colon-cancer cells.**
   **A.** Tumor progression in Balb/c mice subcutaneously injected with CT26 cells (0.5 × 10⁶). After 3 days, treatment with compound 5 (10 mg/kg) or DMSO was given by intraperitoneal injection every other day starting 3 days after the first injection. Two groups of mice (with either DMSO or compound 5 treatment) were treated with anti-PD1 antibody injected subcutaneously every other day. Number of mice for each treatment group = 10.
   **B.** Percentage of Cd11C+ (M1 macrophages) and Cd11C- (M2 macrophages) cells detected in the tumors using flow cytometry.
   **C.** Percentage of CD40+ DCs measured by flow cytometry.
   **D.** Density of CD8+ CD3+ T-cells in tumors from different treatments measured by flow cytometry.

   * p<0.05 against respective controls; # p<0.05 for double treatment against single treatment.

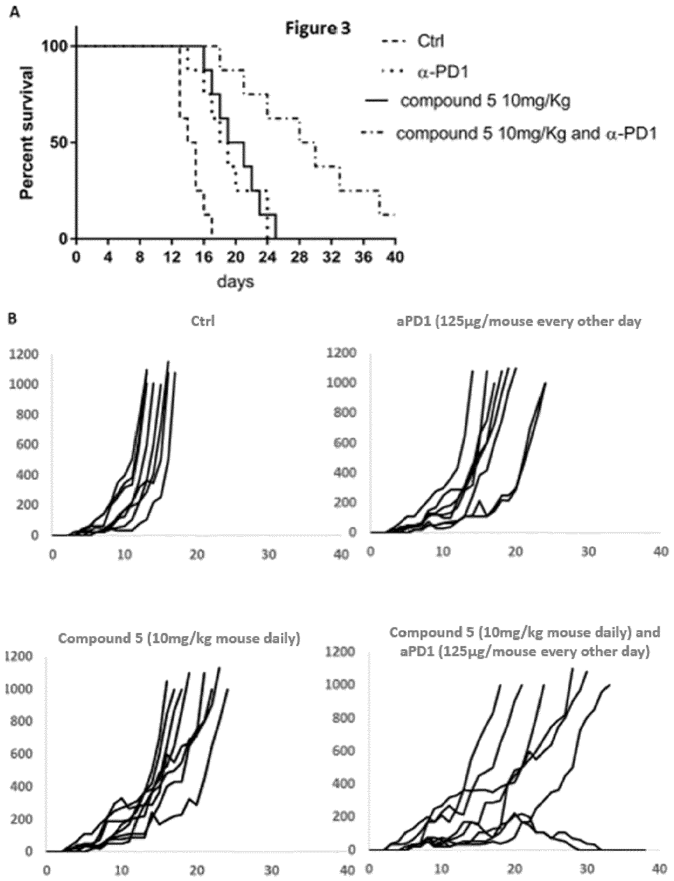

**Figure 3****. Compound 5 treatment is effective when used alone in reducing tumor growth *in vivo* and potentiates the anti-PD1 immune response in C57B16 mice subcutaneously injected with B16 melanoma cells.** B16 cells were injected subcutaneously and the tumors were let to grow until an average volume of 100 mm³ was reached. The mice were then sorted into four groups with similar average tumor volume and standard deviation. Treatment with compound 5 (10 mg/kg) or control (DMSO) was given by intraperitoneal injection every day. Anti-PD1 antibodies (125 µg/mouse) was given every other day alone or combined with daily injection of compound 5. Number of mice for each treatment group = 8.
   **A.** Survival curves. *** p<0.001 against respective controls.
   **B.** Evolution of tumor growth in mm³ for each condition. Evolution in days in the abscissa.

### EXAMPLES

### Example 1: Preparations of compounds

### 1. General procedure for the preparation of compounds of formula 3 wherein R₅ = R₆ =H and X = Cl

0.5 ml of hydrochloric acid 2M, 400mg (2mmol) of 4,7-dichloroquinoline and 2mmol of the amine of formula 4 are added in 15 ml of water. The solution is stirred under reflux for two hours. The colour of the reacting medium changes quickly. After two hours of reaction, the medium is cooled to room temperature. A precipitate corresponding to the expected product appears; it is isolated by filtration and does not require further purification. It is placed in a heat chamber at 60°C to eliminate residual water.

The following compounds 1 to 9 were prepared according to the procedure above:

### 1.1. (4-((3,5-difluorophenyl)amino)-7-chloroquinolinium chloride (compound 1)

Formula: C₁₅H₁₀Cl₂N₂F₂

Exact Mass: 326.0189
Molecular Weight: 327.1558
R=80%

¹H NMR (200 MHz, DMSO-*d6*); δ 14.85 (s, 1H), 11.18 (s, 1H), 8.82 (d, *J* = 9.2 Hz, 1H), 8.64 (d, *J* = 6.9 Hz, 1H), 8.17 (d, *J* = 2.1 Hz, 1H), 7.93 (dd, *J* = 9.2, 2.1 Hz, 1H), 7.43 - 7.24 (m, 3H), 7.10 (d, *J* = 6.9 Hz, 1H).

¹⁹F NMR (188 MHz, DMSO-*d6*); δ 107.96.

¹³C NMR (126 MHz, DMSO-*d6*), δ 163.8 (d, *J=* 15 Hz), 161.9 (d, *J=* 15 Hz), 154.3, 143.9, 140.1 (t), 139.2, 138.5, 127.7, 126.3, 119.4, 116.3, 108.8- 108.3 (2C), 102.7 (t), 101.5.

### 1.2. 4-((4-methylphenyl)amino)-7-chloroquinolinium chloride (compound 2)

Formula: C₁₆H₁₄Cl₂N₂

Exact Mass: 304.0534
Molecular Weight: 305.2020
R = 81 %

¹H NMR (200 MHz, DMSO-*d6*); δ 14.46 (s, 1H), 11.03 (s, 1H), 8.79 (d, J = 9.1 Hz, 1H), 8.50 (d, J = 7.0 Hz, 1H), 8.11 (d, J = 2.1 Hz, 1H), 7.89 (dd, J = 9.1, 2.1 Hz, 1H), 7.38 (d, J = 1.5 Hz, 4H), 6.73 (d, J = 7.0 Hz, 1H), 2.39 (s, 3H).

¹³C NMR (50 MHz, DMSO-^6); δ 155.0, 143.3, 139.1, 138.3, 137.2, 134.3, 130.4 (2C), 127.3, 126.1, 125.4 (2C), 119.2, 115.8, 100.1, 20.7.

### 1.3. 4-((3-trifluoromethylphenyl)amino)-7-chloroquinolinium chloride (compound 3)

Formula: C₁₆H₁₁Cl₂F₃N₂

Exact Mass: 358.0251
Molecular Weight: 359.1732
R = 83 %

¹H NMR (200 MHz, DMSO-*d6*); δ 14.86 (s, 1H), 11.29 (s, 1H), 8.87 (d, J = 9.1 Hz, 1H), 8.60 (d, J = 6.9 Hz, 1H), 8.19 (d, J = 2.1 Hz, 1H), 7.97 - 7.75 (m, 5H), 6.91 (d, J = 6.9 Hz, 1H).

¹⁹F NMR (188 MHz, DMSO-*d6*); δ -60.61, -61.13, -61.21, -61.30, -61.32, -62.06.

¹³C NMR (126 MHz, DMSO-*d6*); δ 154.7, 144.0, 139.2, 138.6, 138.1, 131.3, 130.5 (q), 129.3, 127.7, 126.1, 123.9 (d, J = 3.6 Hz), 123,8 (q, J = 272 Hz), 122.0 (d, J = 3.8 Hz), 119.5, 116.3, 100.7.

### 1.4. 4-((2-methylphenyl) amino)-7-chloroquinolinium chloride (compound 4)

Formula: C₁₆H₁₄Cl₂N₂

Exact Mass: 304.0534
Molecular Weight: 305.2020
R=76%

¹H NMR (200 MHz, DMSO-*d6*); δ 14.71 (s, 1H), 11.08 (s, 1H), 8.89 (d, J = 9.1 Hz, 1H), 8.48 (d, J = 7.0 Hz, 1H), 8.17 (d, J = 2.1 Hz, 1H), 7.89 (dd, J = 9.1, 2.1 Hz, 1H), 7.43 (m, 4H), 6.23 (d, J = 7.0 Hz, 1H), 2.21 (s, 3H).

¹³C NMR (50 MHz, DMSO-*d6*); δ 155.4, 143.4, 138.9, 138.3, 135.2, 135.1, 131.6, 128.7, 127.6, 127.4, 127.3, 125.9, 119.2, 115.4, 99.9, 40.7, 40.3, 39.9, 39.7, 39.5, 39.0, 38.6, 38.2, 17.2.

### 1.5. 4-((4-methoxyphenyl)amino)-7-chloroquinolinium chloride (compound 5)

Formula: C₁₆H₁₄Cl₂N₂O

Exact Mass: 320.0483
Molecular Weight: 321.2010
R=77%

¹H NMR (200 MHz, DMSO-*d6*); δ 14.60 (s, 1H), 11.06 (s, 1H), 8.82 (d, J = 9.2 Hz, 1H), 8.49 (d, J = 7.0 Hz, 1H), 8.14 (d, J = 2.1 Hz, 1H), 7.87 (dd, J = 9.1, 2.1 Hz, 1H), 7.40 (d, J = 8.9 Hz, 2H), 7.13 (d, 2H), 6.65 (d, J = 7.0 Hz, 1H), 3.36 (s, 3H).

¹³C NMR (50 MHz, DMSO-*d6*); δ 158.4, 155.3, 143.0, 138.9, 138.2, 129.3, 127.1, 127.1, 126.0, 119.1, 115.6, 115.1, 99.9, 55.4.

### 1.6. 4-((3-methoxyphenyl)amino)-7-chloroquinolinium chloride (compound 6)

Formula: C₁₆H₁₄Cl₂N₂O

Exact Mass: 320.0483
Molecular Weight: 321.2010
R=64%

¹H NMR (200 MHz, DMSO-*d6*); δ 14.80 (s, 1H), 11.18 (s, 1H), 8.87 (d, J = 9.1 Hz, 1H), 8.52 (d, J = 7.0 Hz, 1H), 8.18 (d, J = 2.1 Hz, 1H), 7.88 (dd, J = 9.2, 2.1 Hz, 1H), 7.49 (t, J = 8.3 Hz, 1H), 7.11 - 6.97 (m, 3H), 6.84 (d, J = 7.0 Hz, 1H), 3.80 (s, 3H).

¹³C NMR (50 MHz, DMSO-*d6*); δ 160.3, 154.8, 143.3, 138.9, 138.3, 138.03, 130.7, 127.3, 126.1, 119.1, 117.3, 115.9, 113.2, 111.0, 100.5, 55.4.

### 1.7. 4-((4-hydroxyphenyl)amino)-7-chloroquinolinium chloride (compound 7)

Formula: C₁₅H₁₂Cl₂N₂O

Exact Mass: 306.0327
Molecular Weight: 307.1740
R=77%

¹H NMR (200 MHz, DMSO-*d6*); δ 14.71 (s, 1H), 11.04 (s, 1H), 9.96 (s, 1H), 8.83 (d, J = 9.2 Hz, 1H), 8.45 (d, J = 7.0 Hz, 1H), 8.15 (d, J = 2.1 Hz, 1H), 7.83 (dd, J = 9.1, 2.0 Hz, 1H), 7.25 (d, J = 8.6 Hz, 2H), 6.95 (d, J = 8.9 Hz, 2H), 6.61 (d, J = 7.0 Hz).

¹³C NMR (50 MHz, DMSO-*d6*); δ 156.9, 155.3, 142.8, 138.8, 138.2, 127.6, 127.1 (3C), 125.9, 118.9, 116.4 (2C), 115.5, 99.8.

### 1.8. 4-((4-hydroxy-3-chlorophenyl)amino)-7-chloroquinolinium chloride (compound 8)

Formula: C₁₅H₁₁Cl₃N₂O

Exact Mass: 339.9937
Molecular Weight: 341.6160
R=85%

¹H NMR (200 MHz, DMSO-*d6*); δ 14.59 (s, 1H), 11.03 (s, 1H), 10.75 (s, 1H), 8.79 (d, J = 9.1 Hz, 1H), 8.50 (d, J = 7.0 Hz, 1H), 8.13 (d, J = 2.1 Hz, 1H), 7.87 (dd, J = 9.1, 2.1 Hz, 1H), 7.50 (d, J = 2.4 Hz, 1H), 7.27 (dd, J = 8.7, 2.4 Hz, 1H), 7.18 (d, J = 8.6 Hz, 1H), 6.69 (d, J = 7.0 Hz, 1H).

¹³C NMR (50 MHz, DMSO-*d6*); δ 155.3, 152.7, 143.1, 138.8, 138.3, 128.4, 127.3, 127.2, 126.0, 125.7, 120.2, 119.1, 117.3, 115.6, 100.2.

### 1.9. 4-((4-trifluoromethylphenyl)amino)-7-chloroquinolinium chloride (compound 9)

Formula: C₁₆H₁₁Cl₂F₃N₂

Exact Mass: 358.0251
Molecular Weight: 359.1732
R=84%

¹H NMR (200 MHz, DMSO-*d6*); δ 14.86 (s, 1H), 11.26 (s, 1H), 8.86 (d, J = 9.1 Hz, 1H), 8.62 (d, J = 6.9 Hz, 1H), 8.17 (d, J = 2.1 Hz, 1H), 8.00 - 7.87 (two overlapping d, 3H), 7.74 (d, J = 8.3 Hz, 2H), 7.05 (d, J = 7.0 Hz, 1H).

¹⁹F NMR (188 MHz, DMSO-*d6*); δ -60.81.

¹³C NMR (50 MHz, DMSO-*d6*); δ 154.3, 143.8, 141.0, 139.1, 138.5, 127.6, 126.9 (4C), 126.2, 125.3 (2C), 119.3, 116.4, 101.0.

### 1.10 7-chloro-N-(4-(difluoromethoxy)phenyl)quinolin-4-amine (compound 34)

Chemical Formula: C₁₆H₁₁ClF₂N₂O

Exact Mass: 320,05
Molecular Weight: 320,72
R=87%

**¹H NMR** (200 MHz, DMSO-*d*6) δ 11.25 (s, 1H), 8.89 (d, *J* = 9.2 Hz, 1H), 8.53 (d, *J* = 7.0 Hz, 1H), 8.18 (d, *J* = 2.1 Hz, 1H), 7.88 (dd, *J=* 9.1, 2.1 Hz, 1H), 7.71 (s, 0H), 7.56 (d, *J* = 8.9 Hz, 2H), 7.43 - 7.32 (m, 2H), 6.98 (s, 0H), 6.75 (d, *J* = 7.0 Hz, 1H).

**¹³C NMR** (101 MHz, DMSO-*d*6) δ 155.2, 149.8, 143.7, 139.1, 138.7, 134.0, 127.6, 127.5, 126.2, 120.4, 119.4, 116.4 (t, *J* = 258.5 Hz), 116.0, 100.4.

**MS:** ESI (*m*/*z*): [M+H]⁻ calcd for C₁₆H₁₁ClF₂N₂O 321.05 found 321.27

**HPLC:** Purity λ₂₅₄: 99.8%, tR: 3.23

### 1.11 7-chloro-N-(4-methoxy-3-(2-methoxyethoxy)phenyl)quinolin-4-amine (compound 35)

Chemical Formula: C₁₉H₁₉ClN₂O₃

Exact Mass: 358,11
Molecular Weight: 358,82
R=75%

**¹H NMR** (500 MHz, DMSO-*d*6) δ 14.48 (s, 1H), 10.95 (s, 1H), 8.77 (d, *J=* 9.1 Hz, 1H), 8.48 (d, *J* = 7.0 Hz, 1H), 8.11 (d, *J* = 2.1 Hz, 1H), 7.86 (dd, *J* = 9.0, 2.1 Hz, 1H), 7.13 (d, *J* = 8.5 Hz, 1H), 7.09 (d, *J* = 2.4 Hz, 1H), 7.00 (dd, *J* = 8.4, 2.4 Hz, 1H), 6.73 (d, *J* = 7.0 Hz, 1H), 4.09 (t, *J* = 4.5 Hz, 2H), 3.83 (s, 3H), 3.67 (d, *J* = 4.7 Hz, 2H), 3.31 (s, 2H).

**¹³C NMR** (50 MHz, DMSO-*d*6) δ 155.2, 148.6, 148.2, 142.9, 138.9, 138.2, 129.4, 127.1, 126.1, 119.0, 117.9, 115.6, 112.4, 110.8, 100.2, 70.2,67.8, 58.2, 55.7,40.7, 40.3, 39.9, 39.5, 39.0, 38.6, 38.2.

**MS:** ESI (*m*/*z*): [M+H] calcd for C₁₉H₁₉ClN₂O₃ 359,11 found 359.20

**HPLC:** Purity λ₂₅₄: 100%, tR: 3.41

### 1.12 7-chloro-N-(3,4-dimethoxyphenyl)quinolin-4-amine (compound 38)

Chemical Formula: C₁₇H₁₅ClN₂O₂

Exact Mass: 314,08
Molecular Weight: 314,77
R = 59%

**¹H NMR** (500 MHz, DMSO-*d6*) δ 14.52 (s, 1H), 10.96 (s, 1H), 8.78 (d, *J* = 9.1 Hz, 1H), 8.48 (d, *J* = 7.0 Hz, 1H), 8.12 (d, *J* = 2.2 Hz, 1H), 7.86 (dd, *J* = 9.1, 2.2 Hz, 1H), 7.13 (d, *J* = 8.6 Hz, 1H), 7.08 (d, *J* = 2.4 Hz, 1H), 7.00 (dd, *J* = 8.5, 2.4 Hz, 1H), 6.73 (d, *J* = 7.0 Hz, 1H), 3.82 (s, 3H), 3.78 (s, 3H).

**¹³C NMR** (50 MHz, DMSO-*d6*) δ 155.2, 149.4, 148.1, 142.9, 138.9, 138.2, 129.5, 127.1, 126.1, 119.0, 117.7, 115.6, 112.2, 109.7, 100.2, 55.7, 40.7, 40.3, 39.9, 39.5, 39.0, 38.6, 38.2.

**MS:** ESI *(m*/*z):* [M+H]⁻ calcd for C₁₇H₁₅ClN₂O₂ 315.08 found 315.13

**HPLC:** Purity λ₂₅₄: 100%, tR: 3.37

### 1.13 7-chloro-N-(2,4-dimethoxyphenyl)quinolin-4-amine (compound 39)

Chemical Formula: C₁₇H₁₅ClN₂O₂

Exact Mass: 314,08
Molecular Weight: 314,77
R=91%

**¹H NMR** (200MHz, DMSO-*d6*) δ: 14.70 (br, 1H), 10.87 (s, 1H), 8.86 (d, *J* = 9.2 Hz, 1H), 8.45 (d, *J* = 7.0 Hz, 1H), 8.20 (d, *J* = 2.1 Hz, 1H), 7.83 (dd, *J* = 9.0, 2.1 Hz, 1H), 7.30 (d, *J* = 8.6 Hz, 1H), 6.81 (d, *J* = 2.5 Hz, 1H), 6.68 (dd, *J* = 8.7, 2.6 Hz, 1H), 6.27 (d, *J* = 7.0 Hz, 1H), 3.84 (s, 3H), 3.77 (s, 3H).

**¹³C NMR** (50MHz, DMSO-*d6*) δ: 160.4, 155.8, 155.3 142.7, 138.9, 138.2, 128.8, 127.2, 126.0, 119.2, 117.4, 115.4, 105.6, 100.6, 99.8, 55.9, 55.6.

**MS:** ESI *(m*/*z):* [M+H]⁻ calcd for C₁₇H₁₅ClN₂O₂ 315.08 found 315.20

**HPLC:** Purity λ₂₅₄: 99.6%, tR: 3.49

### 1.14 7-chloro-N-(2,3-dihydrobenzo[b][1,4]dioxin-6-yl)quinolin-4-amine (compound 40)

Chemical Formula: C₁₇H₁₃ClN₂O₂

Exact Mass: 312,06
Molecular Weight: 312,75
R = 32%

**¹H NMR** (400 MHz, DMSO-*d6*) δ 11.02 (s, 1H), 8.83 (d, *J* = 9.1 Hz, 2H), 8.48 (d, *J* = 7.0 Hz, 2H), 8.16 (d, *J* = 2.2 Hz, 2H), 7.83 (dd, *J* = 9.1, 2.1 Hz, 2H), 7.03 (d, *J* = 8.6 Hz, 2H), 6.99 (d, *J=* 2.4 Hz, 2H), 6.92 (dd, *J* = 8.5, 2.5 Hz, 2H), 6.71 (d, *J* = 6.9 Hz, 2H), 4.30 (s, 9H).

**¹³C NMR** (101 MHz, DMSO) δ 155.1, 144.0, 143.4, 142.8, 139.3, 138.2, 130.0, 127.1, 126.1, 119.4, 118.6, 118.0, 115.8, 114.5, 100.2, 64.2, 64.1, 39.5.

**MS:** ESI *(m*/*z):* [M+H]⁻ calcd for C₁₇H₁₃ClN₂O₂ 313.06 found 313.24

**HPLC:** Purity λ₂₅₄: 100%, tR: 3.09

### 1.15 N-(benzo[d][1,3]dioxol-5-yl)-7-chloroquinolin-4-amine (compound 41)

Chemical Formula: C₁₆H₁₁ClN₂O₂

Exact Mass: 298,05
Molecular Weight: 298,72
R = 17%

**¹H NMR** (400 MHz, DMSO-*d*₆) δ 14.78 (s, 1H), 11.09 (s, 1H), 8.84 (d, *J* = 9.1 Hz, 1H), 8.49 (d, *J* = 7.0 Hz, 1H), 8.17 (d, *J* = 2.1 Hz, 1H), 7.85 (dd, *J* = 9.1, 2.1 Hz, 1H), 7.10 (d, *J* = 3.9 Hz, 1H), 7.08 (d, *J* = 2.2 Hz, 1H), 6.94 (dd, *J* = 8.2, 2.1 Hz, 1H), 6.70 (d, *J* = 7.0 Hz, 1H), 6.14 (s, 2H).

**¹³C NMR** (101 MHz, DMSO) δ 155.4, 148.3, 146.7, 143.2, 139.0, 138.3, 130.6, 127.2, 126.1, 119.3, 119.2, 115.7, 108.9, 107.0, 101.9, 100.3, 39.5.

**MS:** ESI (*m*/*z*): [M+H]⁻ calcd for C₁₆H₁₁ClN₂O₂ 299.05 found 299.18

**HPLC:** Purity λ₂₅₄: 99.0, tR: 3.21

**2. General procedure for the preparation of compounds of formula 3 wherein R₅ is O-alkyl, O-(CH₂)ₙ₁-(cyclic radical), (CH₂)ₙ₁-(cyctic radical), n₁ being 1 to 4, said cyclic radical being chosen from** 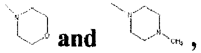 **R₆ = H and X** = **Cl.**

To a solution of compound of formula 6 (1eq) in THF was added slowly sodium methoxide (2eq) or NaH (2eq) follow by the addition of 2-morpholinoethan-1-ol (2eq). The resulting mixture was stirred at reflux until reaction completion, as monitored by TLC. After cooling to r.t, the crude mixture was adsorbed on silica and purified on a column chromatography using methanol/dichloromethane as the eluent (gradient 0/100 to 5/95, v/v).

The following compounds 27 to 33 were prepared according to the procedure above.

### 2.1 7-chloro-2-methoxy-N-(4-methoxyphenyl)quinolin-4-amine (compound 27)

Chemical Formula: C₁₇H₁₅ClN₂O₂

Exact Mass: 314,08
Molecular Weight: 314,77
R = 37%

**¹H NMR** (200 MHz, DMSO-*d*6) δ 8.82 (s, 1H), 8.30 (d, *J* = 8.9 Hz, 1H), 7.66 (d, *J* = 2.2 Hz, 1H), 7.40 (dd, *J* = 8.9, 2.3 Hz, 1H), 7.26 (d, *J* = 8.9 Hz, 2H), 7.01 (d, *J* = 8.9 Hz, 2H), 5.96 (s, 1H), 3.85 (s, 3H), 3.78 (s, 3H).

**¹³C NMR** ¹³C NMR (50 MHz, DMSO-*d*6) δ 164.2, 156.5, 151.3, 148.0, 134.1, 132.4, 125.8, 123.9, 122.6, 116.3, 114.6, 88.6, 55.2, 52.7.

**MS:** ESI (*m*/*z*): [M+H]⁻ calcd for C₁₇H₁₅ClN₂O₂ 315.08 found 315.20

**HPLC:** Purity λ₂₅₄: 97.7%, tR: 3.74

### 2.2 7-chloro-N-(3,4-dimethoxyphenyl)-2-methoxyquinolin-4-amine (compound 33)

Chemical Formula: C₁₈H₁₇ClN₂O₃

Exact Mass: 344,09
Molecular Weight: 344,80
R=34%

**¹H NMR** (200 MHz, DMSO-*d*6) δ 8.80 (s, 1H), 8.28 (d, *J* = 8.9 Hz, 1H), 7.64 (d, *J* = 2.2 Hz, 1H), 7.38 (dd, *J* = 8.8, 2.3 Hz, 1H), 6.99 (d, *J* = 8.5 Hz, 1H), 6.93 - 6.81 (m, 2H), 6.04 (s, 1H), 3.84 (s, 3H), 3.76 (s, 3H), 3.73 (s, 3H).

**¹³C NMR** (50 MHz, DMSO-*d*6) δ 164.2, 151.1, 149.3, 148.0, 146.1, 134.1, 132.8, 125.8, 123.9, 122.6, 116.3, 116.2, 112.3, 108.9, 88.9, 55.7, 55.5, 52.7.

**MS:** ESI (*m*/*z*): [M+H]⁻ calcd for C₁₈H₁₇ClN₂O₃ 345.09 found 345.20

**HPLC:** Purity λ₂₅₄: 97.2%, tR: 3.57

### 2.3 7-chloro-N-(3,4-dimethoxyphenyl)-2-(2-morpholinoethoxy)quinolin-4-amine (compound 32)

Chemical Formula: C₂₃H₂₆ClN₃O₄

Exact Mass: 443,16
Molecular Weight: 443,93
R = 37%

**¹H NMR** (200 MHz, DMSO-*d*6) δ 8.83 (s, 1H), 8.29 (d, *J* = 9.0 Hz, 1H), 7.63 (d, *J* = 2.2 Hz, 1H), 7.40 (dd, *J* = 8.9, 2.2 Hz, 1H), 7.02 (d, *J* = 8.5 Hz, 1H), 6.92 (d, *J* = 2.3 Hz, 1H), 6.87 (dd, *J* = 8.3, 2.4 Hz, 1H), 5.99 (s, 1H), 4.40 (t, *J* = 5.8 Hz, 2H), 3.78 (s, 3H), 3.75 (s, 2H), 3.54 (t, *J* = 4.7 Hz, 5H), 2.63 (t, *J* = 5.8 Hz, 2H), 2.46 - 2.35 (m, 4H).

**¹³C NMR** (50 MHz, DMSO-*d*6) δ 163.8, 151.4, 149.3, 147.9, 146.2, 134.2, 132.7, 125.8, 123.9, 122.6, 116.5, 116.2, 112.3, 109.2, 89.0, 66.1, 62.3, 56.9, 55.6, 55.5, 53.5.

**MS:** ESI (*m*/*z*): [M+H]⁻ calcd for C₂₃H₂₆ClN₃O₄ 444.16 found 444.0

**HPLC:** Purity λ₂₅₄: 99.5%, tR: 3.12

**3. General procedure for the preparation of compounds of formula 3 wherein R₅ is cyclic radical chosen from**

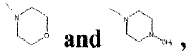

**R₆=H and X = Cl.**

A solution of compound of formula 6 (1eq) was stirred in morpholine (or N-methylpiperazine) 0.5 mmol/mL at 90°C until reaction completion, as monitored by TLC. After cooling to room temperature, the crude mixture is adsorbed on silica and purified on a column using methanol/dichloromethane as the eluent (gradient 0/100 to 5/95, v/v).

The following compounds 28 to 31 were prepared according to the general procedure described above.

### 3.1 7-chloro-N-(4-methoxyphenyl)-2-morpholinoquinolin-4-amine (compound 30)

Chemical Formula: C₂₀H₂₀ClN₃O₂

Exact Mass: 369,12
Molecular Weight: 369,85
R=61%

**¹H NMR** (200 MHz, DMSO-*d*6) δ 8.60 (s, 1H), 8.16 (d, *J* = 8.9 Hz, 1H), 7.46 (d, *J* = 2.2 Hz, 1H), 7.27 (d, *J* = 8.9 Hz, 1H), 7.19 (dd, *J* = 8.8, 2.2 Hz, 1H), 7.04 - 6.95 (m, 2H), 6.24 (s, 1H), 3.77 (s, 3H), 3.64 (t, *J* = 4.7 Hz, 4H), 3.43 (d, *J* = 4.9 Hz, 4H).

**¹³C NMR** (50 MHz, DMSO-*d*6) δ 159.1, 156.0, 149.8, 149.2, 133.8, 132.9, 125.2, 125.0, 123.7, 120.6, 114.6, 114.6, 86.6, 66.0, 55.2, 44.8.

**MS:** ESI *(m*/*z):* [M+H]⁻ calcd for C₂₀H₂₀ClN₃O₂370.12 found 370.27

**HPLC:** Purity λ₂₅₄: 98.2%, tR: 3.36

### 3.2 7-chloro-N-(3,4-dimethoxyphenyl)-2-morpholinoquinolin-4-amine (compound 28)

Chemical Formula: C₂₁H₂₂ClN₃O₃

Exact Mass: 399,13
Molecular Weight: 399,88
R=56%

**¹H NMR** (200 MHz, DMSO-*d*6) δ 8.62 (s, 1H), 8.16 (d, *J* = 8.9 Hz, 1H), 7.47 (d, *J* = 2.2 Hz, 1H), 7.19 (dd, *J* = 8.8, 2.2 Hz, 1H), 7.04 - 6.93 (m, 2H), 6.86 (dd, *J* = 8.4, 2.3 Hz, 1H), 6.37 (s, 1H), 3.76 (s, 3H), 3.74 (s, 3H), 3.65 (t, *J* = 4.6 Hz, 4H), 3.45 (d, *J* = 4.8 Hz, 4H).

**¹³C NMR** ¹³C NMR (50 MHz, DMSO-*d*6) δ 159.1, 149.5, 149.3, 149.2, 145.5, 133.8, 133.3, 125.0, 123.7, 120.6, 115.2, 114.6, 112.3, 108.0, 87.1, 66.0, 55.6, 55.5, 44.8.

**MS:** ESI (*m*/*z*): [M+H]⁻ calcd for C₂₁H₂₂ClN₃O₃ 400.13 found 400.27

**HPLC:** Purity λ₂₅₄: 98.2%, tR: 3.36

### 3.3 7-chloro-N-(4-methoxyphenyl)-2-(4-methylpiperazin-1-yl)quinolin-4-amine (compound 31)

Chemical Formula: C₂₁H₂₃ClN₄O

Exact Mass: 382,16
Molecular Weight: 382,89
R = 57%

**¹H NMR** (200 MHz, DMSO-*d*6) δ 8.56 (s, 1H), 8.14 (d, *J* = 8.9 Hz, 1H), 7.44 (d, *J* = 2.2 Hz, 1H), 7.26 (d, *J* = 8.7 Hz, 2H), 7.17 (dd, *J* = 8.8, 2.2 Hz, 1H), 6.99 (d, *J* = 8.8 Hz, 2H), 6.24 (s, 1H), 3.77 (s, 3H), 3.46 (d, *J=* 4.7 Hz, 4H), 2.34 (d, *J* = 5.0 Hz, 4H), 2.17 (s, 3H).

**¹³C NMR** (50 MHz, DMSO-*d*6) δ 158.9, 156.0, 149.7, 149.4, 133.8, 132.9, 125.1, 124.9, 123.6, 120.4, 114.6, 114.4, 86.8, 55.2, 54.4, 45.7, 44.3.

**MS:** ESI *(m*/*z):* [M+H]⁻ calcd for C₂₁H₂₃ClN₄O 383.16 found 383.20

**HPLC:** Purity λ₂₅₄: 99.5%, tR: 2.76

### 3.4 7-chloro-N-(3,4-dimethoxyphenyl)-2-(4-methylpiperazin-1-yl)quinolin-4-amine (compound 29)

Chemical Formula: C₂₂H₂₅ClN₄O₂

Exact Mass: 412,17
Molecular Weight: 412,92
R = 58%

**¹H NMR** (200 MHz, DMSO-*d*6) δ 8.58 (s, 1H), 8.14 (d, *J* = 8.9 Hz, 1H), 7.45 (d, *J* = 2.2 Hz, 1H), 7.17 (dd, *J* = 8.8, 2.2 Hz, 1H), 7.04 - 6.93 (m, 2H), 6.86 (dd, *J* = 8.4, 2.3 Hz, 1H), 6.39 (s, 1H), 3.76 (s, 3H), 3.74 (s, 3H), 3.48 (t, *J* = 4.7 Hz, 4H), 2.35 (d, *J* = 4.9 Hz, 4H), 2.17 (s, 3H).

**¹³C NMR** (50 MHz, DMSO-*d*6) δ 158.8, 156.1, 149.4, 149.1, 145.4, 133.8, 133.4, 124.9, 123.6, 120.4, 115.1, 114.5, 112.4, 107.9, 87.3, 55.6, 55.4, 54.4,45.7, 44.3.

**MS:** ESI (*m*/*z*): [M+H]⁻ calcd for C₂₂H₂₅ClN₄O₂413.17 found 413.13

**HPLC:** Purity λ₂₅₄: 98.8%, tR: 2.71

### 4. General procedure for the preparation of compounds of formula 3 wherein R₅ = R₂ = H, R₆= Cl.

The following compounds were prepared using the same procedures as compound of formula (3), or alternatively by microwave irradiation at 80°C for 1h of a mixture of 4,6-dichloroquinoline and amine in ethanol. Then, after cooling at r.t ethyl acetate was added and the resulting precipitate was collected, wash with ethyl acetate and diethyl ether to afford pure product without any further purifications.

The following compounds 36 to 37 were prepared according to the procedure above.

### 4.1 6-chloro-N-(2,4-dimethoxyphenyl)quinolin-4-amine (compound 37)

Chemical Formula: C₁₇H₁₅ClN₂O₂

Exact Mass: 314,08
Molecular Weight: 314,77
R = 58%

**¹H NMR** (200MHz, CDCl₃) δ 14.90 (br, 1H), 10.47 (s, 1H), 9.19 (d, *J* = 2.0 Hz, 1H), 8.19 (d, *J* = 9.0 Hz, 1H), 7.98 (d, *J* = 6.9 Hz, 1H), 7.57 (dd, *J* = 9.0, 1.9 Hz, 1H), 7.30-7.26 (m, 1H), 6.39-6.34 (m, 2H), 6.22 (d, *J* = 6.9 Hz, 1H), 3.74 (s, 3H), 3.64 (s, 3H).

**¹³C NMR** (50MHz, CDCl₃) δ 160.8, 155.5, 155.3, 140.4, 137.0, 133.8, 132.9, 128.7, 123.9, 122.3, 118.3, 117.7, 105.2, 100.6, 99.6, 55.7.

**MS:** ESI (*m*/*z*): [M+H]⁻ calcd for C₁₇H₁₅ClN₂O₂ 315.77 found 315.13

**HPLC:** Purity λ₂₅₄: 100%, tR: 3.52

### 4.2 6-chloro-N-(4-(trifluoromethoxy)phenyl)quinolin-4-amine (compound 36)

Chemical Formula: C₁₆H₁₀ClF₃N₂O

Exact Mass: 338,04
Molecular Weight: 338,71
R= 71%

**¹H NMR** (200 MHz, DMSO-*d*6) δ 15.22 (s, 1H), 11.33 (s, 1H), 9.13 (d, *J* = 2.1 Hz, 1H), 8.55 (d, *J* = 6.9 Hz, 1H), 8.19 (d, *J* = 9.0 Hz, 1H), 8.06 (dd, *J* = 9.1, 2.0 Hz, 1H), 7.61 (q, *J* = 8.9 Hz, 4H), 6.88 (d, *J* = 6.9 Hz, 1H).

¹³**C NMR** (101 MHz, DMSO-*d*6) δ 154.1, 146.7, 143.1, 137.0, 136.3, 134.0, 131.7, 127.2, 123.4, 122.5, 122.4, 120.0 (q, *J* = 256.7 Hz), 118.3, 100.5.

**MS:** ESI (*m*/*z*): [M+H]⁻ calcd for C₁₆H₁₀ClF₃N₂O 339.04 found 339.27

**HPLC:** Purity λ254: 99.8%, tR: 3.96

### Example 2: Preparations of compounds of formula 2

### 1. General procedure for the preparation of compounds of formula 2 wherein R₅ = R₆ = H and X= NR₃R₄

100 mg of the compound of formula 3, 1.2 equivalents of the amine of formula 5, 2% molar of [Pd(acac)(IPr*)Cl] and 3 equivalents of tBuOK are placed in a screw cap vial fitted with a septum, under an argon atmosphere before adding 1ml of degassed 1,4-dioxane. The reacting medium is heated at 110°C for 24hours. Upon return to room temperature, the crude mixture is adsorbed on silica and purified on a column using methanol/dichloromethane as the eluent (gradient 0/100 to 5/95, v/v).

The following compounds 10, 12-13, 16, 18-19, 23, 25 and 26 were prepared according to the general procedure described above.

### 1.1 N-(3,5-difluorophenyl)-7-morpholinoquinolin-4-amine (compound 10)

Formula: C₁₉H₁₇F₂N₃O

Exact Mass: 341.1340
Molecular Weight: 341.3618
R = 46 %

¹H NMR (200 MHz, DMSO-*d6*); δ 9.14 (br, 1H), 8.45 (d, J = 5.2 Hz, 1H), 8.13 (d, J = 9.3 Hz, 1H), 7.43 (dd, J = 9.3, 2.6 Hz, 1H), 7.16 (d, J = 2.5 Hz, 1H), 7.06-6.92 (m, 3H), 6.82 (tt, J = 9.3, 2.3 Hz, 1H), 3.79 (t, J = 4.5 Hz, 4H), 3.29 (t, J = 4.5 Hz, 4H).

¹³C NMR (50 MHz, DMSO-*d6*); δ 165.6-165.2 (d), 160.7-160.4 (d), 151.7, 150.5, 150.4, 145.3, 122.8, 116.6, 113.9, 109.6, 102.7 (2C), 102.1, 96.9 (t, J = 26,2 Hz, 2C), 65.9 (2C), 47.9 (2C).

¹⁹F NMR (188 MHz, DMSO-*d6*); δ -109.35, -109.40, -109.44.

HRMS-ESI (m/z) : [M+H]⁺ calcd for C₁₉H₁₈F₂N₃O, 342.1340; found : 342.1416.

### 1.2. N-(o-tolyl)-7-morpholinoquinolin-4-amine (compound 12)

Formula: C₂₀H₂₁N₃O

Exact Mass: 319.1685
Molecular Weight: 319.4080
R = 60 %

¹H NMR (200 MHz, CDCl₃); δ 8.38 (d, J = 5.4 Hz, 1H), 7.85 (d, J = 9.2 Hz, 1H), 7.38 (d, J = 2.4 Hz, 1H), 7.36-7.19 (m, 5H), 6.47 (s, 1H), 6.33 (d, J = 5.4 Hz, 1H), 3.92 (t, J = 4.7 Hz, 4H), 3.34 (d, J = 4.9 Hz, 4H), 2.27 (s, 3H).

¹³C NMR (50 MHz, CDCl₃); δ 152.2, 150.2 149.6, 148.9, 137.8, 133.8, 131.5, 127.3, 126.4, 125.8, 121.1, 116.9, 112.9, 110.7, 100.4, 66.8 (2C), 48.9 (2C), 17.9.

HRMS-ESI (m/z) : [M+H]⁺ calcd for C₂₀H₂₂N₃O, 320.1760; found : 320.1757.

### 1.3. N-(4-methoxyphenyl)-7-morpholinoquinolin-4-amine (compound 13)

Formula: C₂₀H₂₁N₃O₂

Exact Mass: 335.1634
Molecular Weight: 335.4070
R=68%

¹H NMR (200 MHz, CDCl₃); δ 8.38 (d, J = 5.6 Hz, 1H), 7.80 (d, J = 8.9 Hz, 1H), 7.34 - 7.21 (m, 4H), 6.97 (d, J = 8.6 Hz, 2H), 6.55 (d, J = 5.5 Hz, 1H), 3.92 (t, J = 4.7 Hz, 4H), 3.85 (s, 3H), 3.33 (t, J = 4.8 Hz, 4H).

¹³C NMR (50 MHz, DMSO-*d6*); δ 156.1, 151.4, 150.4, 150.2, 148.6, 133.0, 125.3, 122.6, 115.8, 114.5 (2C), 112.7, 109.8 (2C), 98.6, 65.9 (2C), 55.2 (2C), 48.0.

HRMS-ESI (m/z) : [M+H]⁺ calcd for C₂₀H₂₂N₃O₂ 336.1709; found : 336,1706.

### 1.4. N⁴, N⁷-bis(4-methoxyphenyl)quinolin-4,7-diamine (compound 16)

Formula: C₂₃H₂₁N₃O₂

Exact Mass: 371.1634
Molecular Weight: 371.4400
R= 17 %

¹H NMR (500 MHz, MeOD); δ 8.10 (d, J = 9.2 Hz, 1H), 8.02 (d, J = 6.2 Hz, 1H), 7.28 - 7.23 (m, 2H), 7.23-7.19 (m, 2H), 7.16 (dd, J = 9.2, 2.2 Hz, 1H), 7.11 (d, J = 2.3 Hz, 1H),7.03-6.98 (m, 2H), 6.96-6.92 (m, 2H), 6.42 (d, J = 6.2 Hz, 1H), 3.83 (s, 3H), 3.80 (s, 3H).

¹³C NMR (126 MHz, MeOD); δ 159.9, 158.1, 154.3, 151.1, 148.0, 147.2, 135.8, 133.4, 128.1 (2C), 125.1 (2C), 124.6, 118.9, 116.3 (2C), 116.2 (2C), 112.7, 103.4, 99.7, 56.1, 56.4.

HRMS-ESI (m/z) : [M+H]⁺ calcd for C₂₃H₂₂N₃O₂, 372.1709 ; found : 372.1706.

### 1.5. N⁴-(3,5-difluorophenyl)-N⁷-(4-methoxyphenyl)quinolin-4,7-diamine (compound 18)

Formula: C₂₂H₁₇F₂N₃O

Exact Mass: 377.1340
Molecular Weight: 377.3948
R=24%

¹H NMR (200 MHz, DMSO-*d6*); δ 9.30 (br s, 1H), 8.41 (s, 1H), 8.37 (d, J = 5.3 Hz, 1H), 8.08 (d, J = 8.9 Hz, 1H), 7.22 (d, J = 3.2 Hz, 2H), 7.20-7.14 (m, 2H), 6.99 (d, J = 3.2 Hz, 2H), 6.96-6.90 (m, 3H), 6.82 (tt, J = 9.4, 2.3 Hz, 1H), 3.75 (s, 3H).

¹⁹F NMR (188 MHz, DMSO-*d6*); δ -109.62, -109.67, -109.72.

¹³C NMR (50 MHz, DMSO-*d6*); δ 165.6, 160.7, 154.7, 150.4, 149.9, 146.72, 145.7, 134.6, 123.2, 121.9 (2C), 117.2, 114.6 (2C), 113.5, 106.3 (2C), 102.7, 102.3, 102.1, 96.8, 55.2.

HRMS-ESI (m/z) : [M+H]⁺ calcd for C₂₂H₁₈F₂N₃O, 378.1412; found : 378.1415.

### 1.6. 2-chloro-4-((4-((3,5-difluorophenyl)amino)quinolin-7-yl)amino)phenol(compound 19)

Formula: C₂₁H₁₄ClF₂N₃O

Exact Mass: 397.0793
Molecular Weight: 397.8098
R=65%

¹H NMR (500 MHz, DMSO-*d6*); δ 9.90 (s, 1H), 9.09 (s, 1H), 8.39 (s, 1H), 8.37 (s, 1H), 8.07 (d, J = 9.1 Hz, 1H), 7.22 (d, J = 2.4 Hz, 1H), 7.19 (d, J = 2.6 Hz, 1H), 7.16 (dd, J = 9.2, 2.5 Hz, 1H), 7.07 (dd, J = 8.7, 2.7 Hz, 1H), 7.00 - 6.91 (m, 4H), 6.80 (tt, J = 9.6, 2.6 Hz, 1H).

¹³C NMR (126 MHz, DMSO-*d6*) δ 164.1, 163.9, 162.2, 162.1, 153.1, 150.8, 148.4, 146.2, 134.3, 133.1, 123.4, 122.9, 122.9, 121.7, 120.7, 119.8, 117.4, 117.3, 115.8, 115.8, 113.9, 113.5, 107.4, 102.6, 102.5, 102.2, 97.0, 97.0, 96.8, 96.6.

HRMS-ESI (m/z) : [M+H]⁺ calcd for C₂₁H₁₅ClF₂N₃O, 398.0866; found : 398.0866.

### 1.7. N⁴-(o-tolyl)-N⁷-(3-methoxyphenyl)quinolin-4,7-diamine (compound 23)

Formula: C₂₃H₂₁N₃O

Exact Mass: 355.1685
Molecular Weight: 355.4410
R=88%

¹H NMR (200 MHz, CDCl₃); δ 8.35 (d, J = 5.5 Hz, 1H), 7.87 (d, J = 9.0 Hz, 1H), 7.62 (d, J = 2.4 Hz, 1H), 7.36-7.13 (m, 6H), 6.82 (ddd, J = 7.9, 2.0, 0.7 Hz, 1H), 6.77 (t, J = 2.2 Hz, 1H), 6.58 (ddd, J = 8.1, 2.3, 0.7 Hz, 1H), 6.31 (d, J = 5.4 Hz, 1H), 6.19 (s, 1H), 3.80 (s, 3H), 2.27 (s, 3H).

¹³C NMR (126 MHz, MeOD); 162.6, 157.9, 151.94, 150.3, 149.5, 142.9, 136.3, 131.6, 124.8 (2C), 124.3, 118.9, 116.9, 116.1 (2C), 114.1, 111.7, 110.5, 105.7, 101.0, 56.4, 56.2.

HRMS-ESI (m/z) : [M+H]⁺ calcd for C₂₃H₂₂N₃O, 356.1757, found : 356.1764.

### 1.8. N⁴-(3-methoxyphenyl)-N⁷-(4-methoxyphenyl)quinolin-4,7-diamine (compound 25)

Formula: C₂₃H₂₁N₃O₂

Exact Mass: 371.1634
Molecular Weight: 371.4400
R = 58 %

¹H NMR (500 MHz, MeOD); δ 8.14 (d, J = 5.8 Hz, 1H), 8.09 (d, J = 9.1 Hz, 1H), 7.32 (t, J = 8.1 Hz, 1H), 7.24 - 7.20 (m, 2H), 7.19 (d, J = 2.3 Hz, 1H), 7.16 (dd, J = 9.1, 2.4 Hz, 1H), 6.96 - 6.92 (m, 3H), 6.91 (t, J = 2.2 Hz, 1H), 6.77 (ddd, J = 8.3, 2.5, 0.9 Hz, 1H), 6.75 (d, J = 5.9 Hz, 1H), 3.81 (s, 3H), 3.80 (s, 3H).

¹³C NMR (126 MHz, MeOD); δ 162.6, 157.9, 151.9, 150.28, 149.5, 142.9, 136.3, 131.6, 124.8 (2C), 124.3, 118.9, 116.9, 116.1 (2C), 114.1, 111.7, 110.5, 105.7, 101.0, 56.4, 56.2.

HRMS-ESI (m/z) : [M+H]⁺ calcd for C₂₃H₂₂N₃O₂, 372.17093, found : 372.17065.

### 1.9 N⁴-(3,5-difluorophenyl)-N⁷-(3-methoxyphenyl)quinolin-4,7-diamine (compound 26)

Formula: C₂₂H₁₇F₂N₃O

Exact Mass: 377.1340
Molecular Weight: 377.3948
R=23%

¹H NMR (500 MHz, MeOD); δ 8.32 (d, J = 5.4 Hz, 1H), 8.05 (d, J = 9.1 Hz, 1H), 7.52 (d, J = 2.3 Hz, 1H), 7.27 (dd, J = 9.1, 2.4 Hz, 1H), 7.21 (t, J = 8.1 Hz, 1H), 6.98 (d, J = 5.5 Hz, 1H), 6.94 - 6.89 (m, 2H), 6.85 (ddd, J = 8.0, 2.1, 0.9 Hz, 1H), 6.83 (t, J = 2.2 Hz, 1H), 6.62 (tt, J = 9.2, 2.3 Hz, 1H), 6.56 (ddd, J = 8.2, 2.5, 0.9 Hz, 1H), 3.79 (s, 3H).

¹³C (126 MHz, MeOD): δ 165.19 (dd, J = 245, 15 Hz), 162.2, 151.4 , 151.0 , 148.9 , 147.5 , 145.5 (t, J = 13 Hz), 144.7 , 131.0 , 123.8 , 119.7, 115.6, 112.8, 109.1, 108.4, 106.0, 104.6 (dd, J = 14, 7 Hz), 103.0 , 98.8 (t, J = 26 Hz), 55.6.

HRMS-ESI (m/z) : [M+H]⁺ calcd for C₂₂H₁₈F₂N₃O, 378.1412; found : 378.1414. HPLC: Purity λ₂₅₄: 96%, tR: 3.94

### Example 3: In vitro activity of the compounds

### 3.1 Ability to decrease EZH2 mRNA level in A375 (determination of dose-response)

Typically, 30000-50000 cells were plated in six-well culture plates and incubated 24 h later with increasing concentration of NIK inhibitors or vehicle. Cells were incubated with the inhibitors for 4 days prior to harvesting for RNA extraction and PCR. Total RNA was isolated from cells using the RNAeasy minikit (Qiagen) according to the manufacturer's procedure. Reverse transcription was performed using the AMV reverse transcription system (Promega), and quantitative PCR was performed with Power Sybr green (Applied Biosystems, Life Technologies, Grand Island, NY) following the manufacturer's instructions. The PCR was carried out on a Step One plus Real-Time PCR system (Applied Biosystems). All analyses were done in triplicate, and a melting curve analysis was performed to control for product quality and specificity. Expression levels were calculated using the comparative method of relative quantification, with SB34 as a normalizer. The data were analyzed for statistical significance using Student's t-test. The results are presented as the mean ± SEM relative to the control. Primers were obtained from primer bank or primer depot (http://pga.mgh.harvard.edu/primerbank/, https://primerdepot.nci.nih.gov), and their specificity was verified using primer blast (http://www.ncbi.nlm.nih.gov/tools/primer-blast/).

The following results were obtained:
Here IC50 represents the concentration necessary to obtain 50% of EZH2 transcription activity.

| **Compounds tested** | **IC50 (µM)** |
|---|---|
| **5** | **5** |
| **13** | **15** |
| **2** | **10** |
| **6** | **15** |
| **8** | **15** |

By decreasing EZH2 transcription, NIK inhibitors restore senescence in treated cancer cells. A simple beta-galactosidase assay using increasing doses of the compounds allows assessing the potential good candidate. Then the best candidates are tested to study their capacity to decrease EZH2 transcription and concomitantly increasing the expression of CDKN1A and lFN-γ.

### 3.2 A375 cell proliferation assay

Cells were counted using a Malassez chamber. The average and standard deviation were calculated from triplicate experiments. Antiproliferative effects were evaluated by trypan blue exclusion assay. Briefly, cells were seeded into 12-well plate and grown in media supplemented with indicated compound at 1 or 10µM for 48h or 96h. Cells were washed with PBS and detached by trypsinization, collected and colored by trypan blue. Dead and living cells were counted and proliferation was calculated as follow: Proliferation (%) = [(treated cell number/untreated cells number)* 100].

The following results were obtained.

| **Compounds tested** | **Proliferation (%)** | | | |
|---|---|---|---|---|
| | At 1µM - 48H | At 10µM - 48H | At 1µM - 96H | At 10µM - 96H |
| **5** | 91,0 | 67,5 | 77,1 | 58,1 |
| **19** | 74,7 | 0,4 | 74,9 | 0,0 |
| **8** | 81,3 | 82,1 | 74,0 | 67,5 |
| **7** | 84,6 | 95,2 | 91,1 | 79,4 |
| **26** | 54,9 | 0,4 | 9,9 | 0,0 |
| **38** | 90,8 | 33,5 | 46,9 | 15,7 |
| **39** | 88,3 | 18,1 | 34,3 | 13,6 |
| **35** | 103,7 | 84,7 | 85,3 | 79,9 |
| **37** | 84,0 | 80,5 | 87,4 | 53,0 |
| **36** | 104,70 | 49,70 | 97,74 | 9,89 |
| **27** | 98,66 | 67,79 | 85,59 | 21,75 |
| **28** | 98,4 | 92 | 87,85 | 82,77 |
| **29** | 89,6 | 67,2 | 87,57 | 4,80 |
| **30** | 88 | 87,2 | 73,16 | 37,01 |
| **31** | 106,4 | 37,6 | 83,33 | 3,39 |
| **32** | 96 | 100 | 84,46 | 82,20 |
| **33** | 106,4 | 101,6 | 97,18 | 95,20 |
| **34** | 96 | 77,6 | 98,59 | 37,85 |

These results show that the compounds of the invention are efficient to slow tumor cell proliferation.

### 3.3 Melanocyte and fibroblast viability assay

Cell viability effects were evaluated by trypan blue exclusion assay. Briefly, cells were seeded into 12-well plate and grown in media supplemented with indicated compound at 10µM for 96h. Cells were washed with PBS and detached by trypsinization, collected and colored by trypan blue. Dead and living cells were counted and cell viability was calculated as follow: Viability (%) = 100 - [(dead cell number/total cells)* 100].

The following results were obtained :

| **Compounds tested** | Viability (%) at 10 µM | |
|---|---|---|
| | Fibroblast | Melanocyte |
| **5** | 98,6 | 99,5 |
| **19** | n.d | n.d |
| **8** | 98,6 | 98,0 |
| **7** | 97,7 | 99,1 |
| **26** | n.d | n.d |
| **38** | 93,3 | 99,2 |
| **39** | 100,0 | 99,5 |
| **35** | 99,5 | 99,1 |
| **37** | 94,6 | 98,7 |
| **36** | 92,1 | 73,4 |
| **27** | 93,9 | 76,9 |
| **28** | 95,7 | 99,1 |
| **29** | 41,6 | 91,2 |
| **30** | 96,4 | 94,2 |
| **31** | 29,6 | 33,1 |
| **32** | 99,4 | 98,6 |
| **33** | 97,7 | 60,1 |
| **34** | 98,3 | 98,7 |

| | | |
|---|---|---|
| n.d: non-determined | | |

These results show that the compounds of the invention do not present any toxicity towards normal human fibroblast and melanocyte.

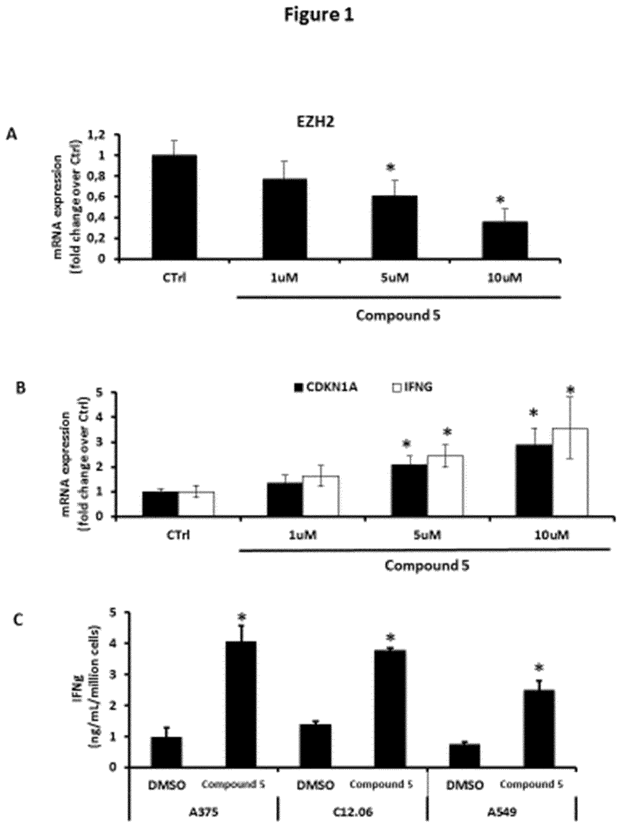

The compounds of the invention, in particular compound **5,** restored senescence in A375 cells with a clear, dose-dependent effect. They also decreased EZH2 transcription in a dose-dependent manner (Figure 1A for compound 5) and concomitantly increased the expression of CDKN1A and IFN-γ (Figure 1B for compound 5). The secretomes of A375, C12.06, and A549 cells treated with compound **5** were studied. The secretomes produced by the compound **5**-treated cells had a clear IFN-γ signature and were similar to those produced by NFkB2-siRNA-treated cells (Figure I C). The effects of compound **5** with those of NFkB2-siRNA were compared on the proliferation of A375, C12.06, and A549 cells. Compared with controls, the treatments had similar inhibitory effects on the proliferation of the cancer cells. Neither treatment had any effect on the proliferation of normal human melanocytes or fibroblasts. The A375, C12.06, and A549 cells treated with compound **5** produced significantly more IFN-γ than control cells . Finally, compound **5** decreased p52, and EZH2 at the protein level (and in some cases also RelB) in each of the cancer cell types. Compound **5** also induced an increase in STAT1 phosphorylation but did not increase cleaved PARP or activate caspase 3, LC3 or CHOP, supporting its specificity.

### Example 4: In vivo activity of the compounds of the invention

In light of the promising in vitro results, the efficacy of compound **5** was tested in vivo for treating solid cancer, either alone or with immune-checkpoint blockers.

### 4.1 In colorectal cancer

### Subcutaneous tumor development

0.5 × 10⁶ cells in 100 µL PBS were subcutaneously injected into 4-wcek-old female C57B1/6 mice (for the B16 experiment) or Balb/c mice (for the CT26 experiments). DZNEP is an inhibitor of the catalytic activity of EZH2 and was used as an additional control.

1 mg/kg DZNEP and 10 mg/kg compound **5** were used for the subsequent experiments. Tridimensional measurements were performed daily, and the tumor volume was expressed as mm³. The tumors were allowed to grow until the total burden per mouse reached 1000 mm³ or until the mice showed > 20% weight loss, at which point the mice were sacrificed. Freshly collected tumors were dissociated and stained for immune-cell infiltrate using a flow cytometer. CD3+ T cells from spleen tissue were negatively selected using myltennyi magnetic columns and put in contact with naive CT26 cells for 24 or 48 h. The CT26 cells were subsequently stained with DAPI (0.5 µg/ml, Molecular Probes, Grand Island, NY, USA). The T-cells were stained with CD8, perforin, and granzyme B antibodies and analyzed immediately by flow cytometry using a MACS-Quant Analyzer (Miltenyi Biotec, Paris, France).

All animal experimentation was carried out in accordance with the Declaration of Helsinki and was reviewed and approved by the French Ethic Committee (Minister of Agriculture, Approval Number Protocol: PEA NCE/2013-73)

### Immune cell staining and analysis

Tumors were extracted and disaggregated using 70 µM strainers. Then, the cells were re-suspended in ice-cold PBS (Life Technologies, 14190169) and incubated for 30 min (4°C in the dark) with one or more of the following monoclonal anti-mouse antibodies, all of which are specific for mouse epitopes, according to the manufacturer's protocol: eFluor^{®} 450-anti-F4/80 (BM8, eBioscience), PE/FITC/APC-anti-CD3 (145-2C11, BD Pharmingen), Pacific Blue-anti-CD4 (RM4-5, BD Pharmingen), PE-Cy7-anti-CD8 (53-6.7, BD Pharmingen), APC-anti-CD11b (M1/70, BD Pharmingen), FITC/PE-anti-CD11c (HL3, BD Pharmingen), APC-Cy7-anti-CD25 (PC61, BD Pharmingen), PE-anti-CD40 (3/23, BD Pharmingen), FITC-anti-CD80 (B7-1, BD Pharmingen), PE-Cy7-anti-CD86 (B7-2, BD Pharmingen), FITC/PE-anti-NK1.1 (PK136, BD Pharmingen), PE-anti-FoxP3 (MF23, BD Pharmingen), APC/PE-anti-IFN-γ (XMG1.2, BD Pharmingen), PE-anti-perforin (eBioOMAK-D, eBioscience), and PE-Cy7-anti-Granzyme B (NGZB RUO, eBioscience). FoxP3 was detected by cell intranuclear staining employing the BD Pharmingen^{™} Mouse Foxp3 Buffer Set according to the manufacturer's instructions. lFN-γ, perforin, and granzyme B were detected by intracellular staining using the BD Cytofix/Cytoperm^{™} Kit following the manufacturer's instructions. Once stained, the cells were washed twice with PBS prior to analysis by flow cytometry. Fluorescence was measured using a MACSQuant^{®} Analyzer (Miltenyi, Paris, France).

### Negative selection

Spleens were extracted and disaggregated, and the cells were resuspended in PBS. T-cells were isolated by CD3 negative selection to avoid activation. For that purpose, the cells were incubated for 15 min (4°C in the dark) with the following mixture of monoclonal FITC-anti-mouse antibodies according to the manufacturer's protocol: TER-119 (TER-119, eBioscience), CD19 (6D5, BD Pharmingen), CD45R/B220 (30-F11, BD Pharmingen), CDllb (M1/70, BD Pharmingen), and CD49b (DX5, BD Pharmingen). Then, the cells were washed and incubated with magnetic anti-FITC MicroBeads for 15 min. The cells were then washed, centrifuged, and resuspended in 500 µL PBS prior to passage through an LS Column (Miltenyi) attached to a MACS^{®} Separator (Miltenyi) followed by 3 mL PBS. The flow-through containing the enriched fraction of CD3 cells was collected and analyzed by flow cytometry to determine the yield of the enrichment (CD3 cells constituted a minimum 70% of the total cells collected).

### Statistical analysis

The majority of the data are presented as the average ± SD of at least three independent experiments, each performed in triplicate. The statistical significance of the experiments was evaluated using an unpaired two-tailed Student's t-test; * indicates a *P* value < 0.05; ** indicates a P value < 0.01. Representative experiments are presented as the average ± SD of the triplicate measurements of an experiment that was reproduced at least two times.

Tests for statistical significance in the animal experiments were performed using the Mann Whitney Wilcoxon test.

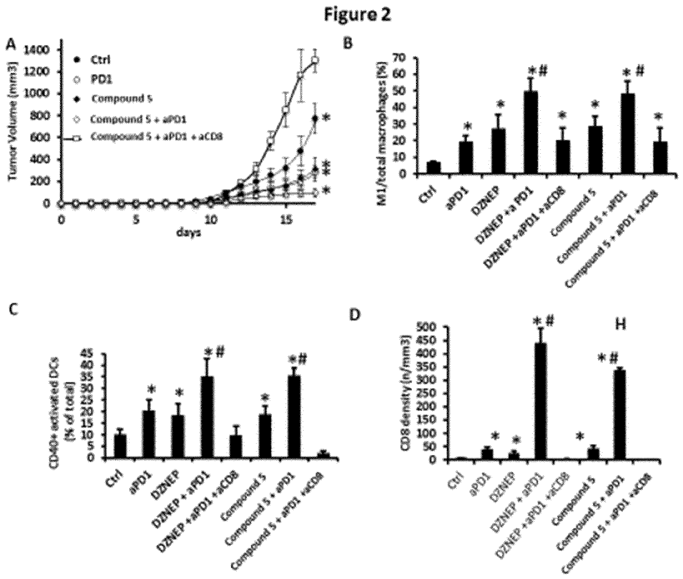

In order to evaluate the new approach in different types of cancer, compound **5** was tested in colorectal cancer cells. CT26 cells were subcutaneously injected into syngeneic mice. After 3 days, the mice were treated intraperitoneally with DZNEP, compound **5,** and anti-PD1 as monotherapies and combined. Additional treatments in which the mice received DZNEP or compound **5** plus anti-PD1 with an anti-CD8 antibody added to neutralize the T-cell response were included. The growth of the CT26 tumors was significantly reduced by each monotherapy (Figures 2A). The combination treatments had a dramatic effect on tumor size, not only reducing the tumor growth but also decreasing the tumor volume in the last days of the experiment. Signs of tumor regression and tumor-free mice were even observed. When anti-CD8 was added to the combination treatment, the growth of the tumors was markedly increased, highlighting the role of immune surveillance in controlling tumor development. The number of M1 macrophages and the percentage of M1 macrophages among the total macrophage population were increased in all the treated mice, especially in the mice that received the combination treatments (Figure 2C). Each of the treatments increased the numbers and activation of DCs and the numbers of CD8+ and CD4+ cells infiltrating the tumors (Figure 2D). The treatments increased the number of NK cells infiltrating the tumors, and the increase was significantly greater for the combination therapies The mice treated with compound **5** showed no clinical signs of toxicity and had similar liver weight and systemic glycemia compared to the other mice. The liver enzymes in the blood of the mice treated with compound **5** remained in the normal range. Finally, CD3+ T-cells were isolated by negative selection from the spleens of the treated mice and cocultured with naive CT26 cells. The T-cells isolated from the mice treated with DZNEP, compound **5,** or the combination therapies were more effective in killing CT26 cells. The CD8+ cells from the treated mice showed higher expression levels of perforin and granzyme B.

These results show not only that compound **5** is well tolerated and effective in reducing tumor growth by itself but also that compound **5** clearly potentiates anti-PD1 treatment. The immune cells that are recruited and activated at the tumor site by compound **5** are able to recirculate and then recognize and efficiently kill new tumor cells.

### 4.2 In melanoma

The inventors then asked whether compound **5** was able to increase the survival rate of mice with already established tumor and, if that was the case, also in this situation was able to synergize with anti-PD1 treatment. They thus decided to use the model B16 melanoma cells in C57B16 mice. B16 cells were again injected subcutaneously and the tumors were let to grow until an average volume of 100 mm3 was reached. The mice were then sorted into four groups with similar average tumor volume and standard deviation and the treatments (control group, compound **5,** anti-PD1 and combination of compound **5** and anti-PD1) applied with the same protocol as in the CT26 experiment. The tumors were allowed to grow until the total burden per mouse reached 1000 mm3, at which point the mouse was sacrificed. The results indicate that even in this case the single treatments (median survival for compound 5 treatment was 20 days from injection while for anti-PD1 was 19 days) were effective in reducing the tumor growth in comparison with the control (median survival was 13 days) (Figure 3A and 3B). The combination of treatments demonstrated a **synergistic effect** with 2 cases of tumor regression and a median survival of 28 days. Moreover, in this latter experiment, no death dependent from compound **5** treatment was observed. Taken globally, these last results thus show not only that compound **5** is well tolerated and effective in reducing tumor growth by itself but also that compound **5** clearly potentiates anti-PD1 treatment in particular in lung-cancer and colon-cancer.

### REFERENCES:

Throughout this application, various references describe the state of the art to which this invention pertains.
1. J. Fu, I. J. Malm, D. K. Kadayakkara, H. Levitsky, D. Pardoll, Y. J. Kim, Preclinical evidence that PD1 blockade cooperates with cancer vaccine TEGVAX to elicit regression of established tumors. Cancer research 74, 4042-4052 (2014).
2. T. Bald, J. Landsberg, D. Lopez-Ramos, M. Renn, N. Glodde, P. Jansen, E. Gaffal, J. Steitz, R. Tolba, U. Kalinke, A. Limmer, G. Jonsson, M. Holzel, T. Tuting, Immune cell-poor melanomas benefit from PD-1 blockade after targeted type 1 IFN activation. Cancer discovery 4, 674-687 (2014).
3. Z. Guo, H. Wang, F. Meng, J. Li, S. Zhang, Combined Trabectedin and anti-PD1 antibody produces a synergistic antitumor effect in a murine model of ovarian cancer. J Transl Med 13, 247 (2015).
4. H. E. Teulings, J. Limpens, S. N. Jansen, A. H. Zwinderman, J. B. Reitsma, P. I. Spuls, R. M. Luiten, Vitiligo-like depigmentation in patients with stage III-IV melanoma receiving immunotherapy and its association with survival: a systematic review and meta-analysis. J Clin Oncol 33, 773-781 (2015).
5. H. E. Teulings, M. Overkamp, E. Ceylan, L. Nieuweboer-Krobotova, J. D. Bos, T. Nijsten, A. W. Wolkerstorfer, R. M. Luiten, J. P. van der Veen, Decreased risk of melanoma and nonmelanoma skin cancer in patients with vitiligo: a survey among 1307 patients and their partners. Br J Dermatol 168, 162-171 (2013).
6. M. Rashighi, P. Agarwal, J. M. Richmond, T. H. Harris, K. Dresser, M. W. Su, Y. Zhou, A. Deng, C. A. Hunter, A. D. Luster, J. E. Harris, CXCL10 is critical for the progression and maintenance of depigmentation in a mouse model of vitiligo. Sci Transl Med 6, 223ra223 (2014).
7. G. M. De Donatis, E. L. Pape, A. Pierron, Y. Cheli, V. Hofman, P. Hofman, M. Allegra, K. Zahaf, P. Bahadoran, S. Rocchi, C. Bertolotto, R. Ballotti, T. Passeron, NF-kB2 induces senescence bypass in melanoma via a direct transcriptional activation of EZH2. Oncogene, (2015).
8. T. W. Kang, T. Yevsa, N. Woller, L. Hoenicke, T. Wuestefeld, D. Dauch, A. Hohmeyer, M. Gereke, R. Rudalska, A. Potapova, M. Iken, M. Vucur, S. Weiss, M. Heikenwalder, S. Khan, J. Gil, D. Bruder, M. Manns, P. Schirmacher, F. Tacke, M. Ott, T. Luedde, T. Longerich, S. Kubicka, L. Zender, Senescence surveillance of pre-malignant hepatocytes limits liver cancer development. Nature 479, 547-551 (2011).

## Claims

1. A compound of general formula (I): 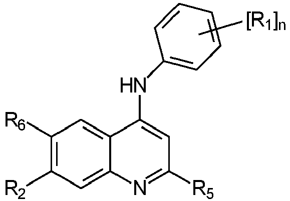 wherein
R₁ is selected from H, alkyl, Halo, OH, O-alkyl, NH₂, NH-alkyl, N-(alkyl)₂, S-alkyl, CF₃, OCF₃, OCF₂H and O(CH₂)ₙ₁O(CH₂)ₙ₂CH₃, n₁ being 1 to 4 and n₂ being 0 to 3,
Halo being selected from Cl, F, Br and I;
n is 1 or 2,
when n is 2, then it is also possible for R₁ to form with the phenyl group at the meta and para positions a dioxolane group or a 1,4 dioxane group,
R₂ is selected from Halo and NR₃R₄, Halo being defined as previously,
R₃ is H and R₄ is phenyl optionally substituted with one or more substituents selected from OH, O-alkyl and Halo,
or R₃ and R₄ together form a (CH₂)₂O(CH₂)₂ chain, that is to say that NR₃R₄ forms a morpholino group 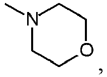
R₅ is selected from H, cyclic radical, O-alkyl, O-(CH₂)ₙ₁-(cyclic radical), (CH₂)ₙ₁-(cyclic radical), n₁ being defined as previously, said cyclic radical being chosen from 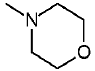 and 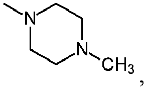
R₆ is selected from H and Halo,
with the proviso that when R₂ is Halo or R₆ is Halo then R₅ is not H,
or a pharmaceutically acceptable salt and/or optical isomer, tautomer, solvate or isotopic variation thereof.

2. A compound according to claim 1 of general formula (2): 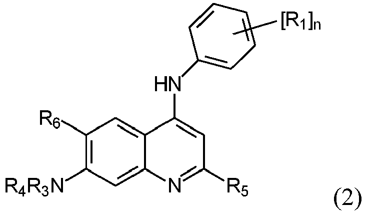
wherein R₁, R₃, R₄, R₅, R₆ and n are as defined in claim 1,
or a pharmaceutically acceptable salt and/or optical isomer, tautomer, solvate or isotopic variation thereof.

3. A compound according to claim 1 of general formula (3): 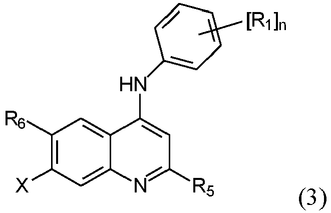
wherein R₁, R₅, R₆, and n are as defined in claim 1 and X is a Halo chosen from Cl, F, Br and I, and with the proviso that R₅ is not H,
or a pharmaceutically acceptable salt and/or optical isomer, tautomer, solvate or isotopic variation thereof.

4. A compound according to any one of claims 1 to 3, wherein R₁ is selected from methyl (CH₃), fluoro (F), chloro (Cl), hydroxy (OH), methoxy (OCH₃), CF₃, OCF₃, OCF₂H and O(CH₂)₂OCH₃, wherein when n is 2 then the substituents R₁ are identical or different.

5. A compound according to any one of claims 1 to 3, wherein when n is 2 then the two R₁ form with the phenyl group at the meta and para positions a dioxolane group or a 1,4 dioxane group.

6. A compound according to any one of claims 1, 2 and 4, wherein R₃ is H and R₄ is phenyl unsubstituted or substituted with one or two groups identical or different group selected from OH, OCH₃ and Halo, preferably chloro.

7. A compound according to any one of claims 1, 2 and 4, wherein R₃ and R₄ together form a (CH₂)₂O(CH₂)₂ chain, that is to say that NR₃R₄ forms a morpholino group.

8. A compound according to claim 1, which is selected from:
- *N*-(3,5-difluorophenyl)-7-morpholinoquinolin-4-amine,
- *N*-(*p*-tolyl)-7-morpholinoquinolin-4-amine,
- *N*-(*o*-tolyl)-7-morpholinoquinolin-4-amine,
- *N*-(4-methoxyphenyl)-7-morpholinoquinolin-4-amine,
- *N*-(3-methoxyphenyl)-7-morpholinoquinolin-4-amine,
- *N*-(3-(trifluoromethyl)phenyl)-7-morpholinoquinolin-4-amine,
- *N⁴*, *N⁷*-bis(4-methoxyphenyl)quinolin-4,7-diamine,
- *N⁴*, *N⁷*-bis(3-methoxyphenyl)quinolin-4,7-diamine,
- *N⁴*-(3,5-difluorophenyl)-*N⁷*-(4-methoxyphenyl)quinolin-4,7-diamine,
-*N⁴*-(3,5-difluorophenyl)-*N⁷*-(3-chloro-4-hydroxyphenyl)quinolin-4,7-diamine,
- *N⁴*-(*p*-tolyl)-*N⁷*-(3-methoxyphenyl)quinolin-4,7-diamine,
- *N⁴*-(*p*-tolyl)-*N⁷*-(4-methoxyphenyl)quinolin-4,7-diamine,
- *N⁴*-(3-(trifluoromethyl)phenyl)-*N⁷*-(4-methoxyphenyl)quinolin-4,7-diamine,
- *N⁴*-(*o*-tolyl)-*N⁷*-(3-methoxyphenyl)quinolin-4,7-diamine,
- *N⁴*-(4-methoxyphenyl)-*N⁷*-(3-methoxyphenyl)quinolin-4,7-diamine,
- *N⁴*-(3-methoxyphenyl)-*N⁷*-(4-methoxyphenyl)quinolin-4,7-diamine,
- *N⁴*-(3,5-difluorophenyl)-*N⁷*-(3-methoxyphenyl)quinolin-4,7-diamine,
- *N*-(4-methoxyphenyl)-7-chloro-2-methoxy-quinolin-4-amine,
- *N*-(3,4-dimethoxyphenyl)-7-chloro-2-morpholino-quinolin-4-amine,
- *N* (3,4-dimethoxyphenyl)-7-chloro-2-(4-methylpiperazin-1-yl)-quinolin-4-amine,
- *N*-(4-methoxyphenyl)-7-chloro-2-morpholino-quinolin-4-amine,
- *N*-(4-methoxyphenyl)-7-chloro-2-(4-methylpiperazin-1-yl)-quinolin-4-amine,
- *N*-(3,4-dimethoxyphenyl)-7-chloro-2-(2-morpholinoethoxy)quinolin-4-amine and
- *N*-(3,4-dimethoxyphenyl)-7-chloro-2-methoxy-quinolin-4-amine;
or a pharmaceutically acceptable salt and/or optical isomer, tautomer, solvate or isotopic variation thereof.

9. A compound according to any one of claims 1 to 8 for use in the treatment of cancer, said cancer being preferably selected from melanoma, bladder, kidney, prostate, colon, lung, breast and blood cancer.

10. A pharmaceutical composition as a combined preparation for simultaneous, separate or sequential use in the treatment of cancer, comprising:
- a compound of general formula (I) 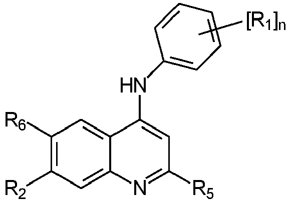 wherein
R₁ is selected from H, alkyl, Halo, OH, O-alkyl, NH₂, NH-alkyl, N-(alkyl)₂, S-alkyl, CF₃, OCF₃, OCF₂H and O(CH₂)ₙ₁O(CH₂)ₙ₂CH₃, n₁ being 1 to 4 and n₂ being 0 to 3,
Halo being selected from Cl, F, Br and I;
n is 1 or 2;
when n is 2, then it is also possible for R₁ to form with the phenyl group at the meta and para positions a dioxolane group or a 1,4 dioxane group,
R₂ is selected from H, Halo and NR₃R₄; Halo being defined as previously,
R₃ is H and R₄ is phenyl optionally substituted with one or more substituents selected from OH, O-alkyl and Halo, or R₃ and R₄ together form a (CH₂)₂O(CH₂)₂ chain,
R₅ is selected from H, cyclic radical, O-alkyl, O-(CH₂)ₙ₁-(cyclic radical), (CH₂)ₙ₁-(cyclic radical), n₁ being defined as previously, said cyclic radical being chosen from 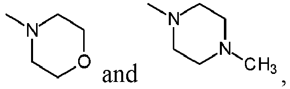
R₆ is selected from H and Halo,
or a pharmaceutically acceptable salt and/or optical isomer, tautomer, solvate or isotopic variation thereof,
- at least one antibody selected from an anti-PD1 antibody, an anti-CTLA4 antibody, an anti-PD-L1 antibody and a mixture of two or more thereof, and
- optionally at least one pharmaceutically acceptable carrier.

11. The pharmaceutical composition for use according to claim 10 wherein compound (I) is selected from:
-*N*-(3,5-difluorophenyl)-7-chloroquinolin-4-amine,
- *N*-(4-methylphenyl)-7-chloroquinolin-4-amine,
- *N*-(3-trifluoromethylphenyl)-7-chloroquinolin-4-amine,
- *N*-(2-methylphenyl)-7-chloroquinolin-4-amine,
- *N*-(4-methoxyphenyl)-7-chloroquinolin-4-amine,
- *N*-(3-methoxyphenyl)-7-chloroquinolin-4-amine,
- *N*-(4-hydroxyphenyl)-7-chloroquinolin-4-amine,
-*N*-(4-hydroxy-3-chlorophenyl)-7-chloroquinolin-4-amine,
-*N*-(4-trifluoromethylphenyl)-7-chloroquinolin-4-amine,
- *N-*(3,5-difluorophenyl)-7-morpholinoquinolin-4-amine,
- *N-*(4-(difluoromethoxy)phenyl)-7-chloro-quinolin-4-amine,
- *N-*(4-methoxy-3-(2-methoxyethoxy)phenyl)-7-chloro-quinolin-4-amine,
- *N*-(4-trifluoromethoxyphenyl)-6-chloroquinolin-4-amine,
- *N*-(2,4-dimethoxyphenyl)-6-chloroquinolin-4-amine,
- *N*-(3,4-dimethoxyphenyl)-7-chloroquinolin-4-amine,
- *N*-(2,4-dimethoxyphenyl)-7-chloroquinolin-4-amine,
- *N*-(*p*-tolyl)-7-morpholinoquinolin-4-amine,
- *N*-(*o*-tolyl)-7-morpholinoquinolin-4-amine,
- *N*-(4-methoxyphenyl)-7-morpholinoquinolin-4-amine,
- N-(3-methoxyphenyl)-7-morpholinoquinolin-4-amine,
- *N*-(3-(trifluoromethyl)phenyl)-7-morpholinoquinolin-4-amine,
- *N⁴*, *N⁷*-bis(4-methoxyphenyl)quinolin-4,7-diamine,
- *N⁴*, *N⁷*-bis(3-methoxyphenyl)quinolin-4,7-diamine,
- *N⁴*-(3,5-difluorophenyl)-*N⁷*-(4-methoxyphenyl)quinolin-4,7-diamine,
-*N⁴*-(3,5-difluorophenyl)-*N⁷*-(3-chloro-4-hydroxyphenyl)quinolin-4,7-diamine,
- *N⁴*-(*p*-tolyl)-*N⁷*-(3-methoxyphenyl)quinolin-4,7-diamine,
- *N⁴*-(*p*-tolyl)-*N⁷*-(4-methoxyphenyl)quinolin-4,7-diamine,
- *N⁴*-(3-(trifluoromethyl)phenyl)-*N⁷*-(4-methoxyphenyl)quinolin-4,7-diamine,
- *N⁴*-(*o*-tolyl)-*N⁷*-(3-methoxyphenyl)quinolin-4,7-diamine,
- *N⁴*-(4-methoxyphenyl)-*N⁷*-(3-methoxyphenyl)quinolin-4,7-diamine,
- *N⁴*-(3-methoxyphenyl)-*N⁷*-(4-methoxyphenyl)quinolin-4,7-diamine,
- *N⁴*-(3,5-difluorophenyl)-*N⁷*-(3-methoxyphenyl)quinolin-4,7-diamine,
- *N*-(4-methoxyphenyl)-7-chloro-2-methoxy-quinolin-4-amine,
- *N*-(3,4-dimethoxyphenyl)-7-chloro-2-morpholino-quinolin-4-amine,
- *N* (3,4-dimethoxyphenyl)-7-chloro-2-(4-methylpiperazin-1-yl)-quinolin-4-amine,
- *N*-(4-methoxyphenyl)-7-chloro-2-morpholino-quinolin-4-amine,
- *N*-(4-methoxyphenyl)-7-chloro-2-(4-methylpiperazin-1-yl)-quinolin-4-amine,
- *N*-(3,4-dimethoxyphenyl)-7-chloro-2-(2-morpholinoethoxy)quinolin-4-amine,
- *N*-(3,4-dimethoxyphenyl)-7-chloro-2-methoxy-quinolin-4-amine,
- 7-chloro-*N*-(2,3-dihydrobenzo[*b*][1,4]dioxin-6-yl)quinolin-4-amine, and
- 7-chloro-*N-*(benzo[d][1,3]dioxol-5-yl)quinolin-4-amine;
or a pharmaceutically acceptable salt and/or optical isomer, tautomer, solvate or isotopic variation thereof.

12. A pharmaceutical composition comprising a compound according to any one of claims 1 to 8 and a pharmaceutically acceptable carrier.

13. The pharmaceutical composition according to claim 12, additionally comprising another anti-cancer drug.

14. The pharmaceutical composition for use according to claim 10 or 11, wherein said cancer is selected from melanoma, bladder, kidney, prostate, colon, lung, breast and blood cancer.

15. A compound of general formula (I): 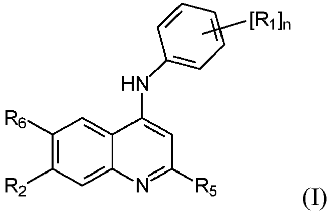 wherein
R₁ is selected from H, Halo, OH, O-alkyl, NH₂, NH-alkyl, N-(alkyl)₂, S-alkyl, OCF₃, OCF₂H and O(CH₂)ₙ₁O(CH₂)ₙ₂CH₃, n₁ being 1 to 4 and n₂ being 0 to 3, Halo being selected from Cl, F, Br and I;
n is 1 or 2,
when n is 2, then it is also possible for R₁ to form with the phenyl group at the meta and para positions a dioxolane group or a 1,4 dioxane group,
R₂ is selected from Halo and NR₃R₄, Halo being defined as previously,
R₃ is H and R₄ is phenyl optionally substituted with one or more substituents selected from OH, O-alkyl and Halo,
or R₃ and R₄ together form a (CH₂)₂O(CH₂)₂ chain, that is to say that NR₃R₄ forms a morpholino group 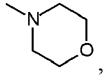
R₅ is selected from H, cyclic radical, O-alkyl, O-(CH₂)ₙ₁-(cyclic radical), (CH₂)ₙ₁-(cyclic radical), n₁ being defined as previously, said cyclic radical being chosen from 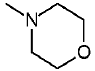 and 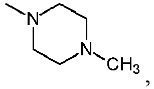
R₆ is selected from H and Halo,
with the proviso that when R₂ is F and R₅ = R₆ = H then R₁ is not 3-Cl; 4-Cl; 3-F; 4-OCH₃; 3-C1 and 4-F; 3-Cl and 4-Cl; or 4-F;
and with the proviso that when R₂ is Cl and R₅ = R₆ = H then R₁ is not 4-OH or 4-Cl;
or a pharmaceutically acceptable salt and/or optical isomer, tautomer, solvate or isotopic variation thereof,
for use in the treatment of cancer.

16. The compound for use according to claim 15, wherein said cancer is selected from melanoma, bladder, kidney, prostate, colon, lung, breast and blood cancer, preferably wherein said cancer is selected from melanoma and colon cancer.

17. The compound for use according to claim 15 or 16, wherein said compound is selected from:
- *N*-(3,5-difluorophenyl)-7-chloroquinolin-4-amine,
- *N*-(4-methoxyphenyl)-7-chloroquinolin-4-amine,
- *N*-(3-methoxyphenyl)-7-chloroquinolin-4-amine,
- *N*-(3,5-difluorophenyl)-7-morpholinoquinolin-4-amine,
- *N*-(4-(difluoromethoxy)phenyl)-7-chloro-quinolin-4-amine,
- *N*-(4-methoxy-3-(2-methoxyethoxy)phenyl)-7-chloro-quinolin-4-amine,
- *N-*(3,4-dimethoxyphenyl)-7-chloroquinolin-4-amine,
- *N*-(2,4-dimethoxyphenyl)-7-chloroquinolin-4-amine,
- *N*-(4-methoxyphenyl)-7-morpholinoquinolin-4-amine,
- *N*-(3-methoxyphenyl)-7-morpholinoquinolin-4-amine,
- *N⁴*, *N⁷*-bis(4-methoxyphenyl)quinolin-4,7-diamine,
- *N⁴*, *N⁷*-bis(3-methoxyphenyl)quinolin-4,7-diamine,
- *N⁴*-(3,5-difluorophenyl)-*N*⁷-(4-methoxyphenyl)quinolin-4,7-diamine,
-*N⁴*-(3,5-difluorophenyl)-*N⁷*-(3-chloro-4-hydroxyphenyl)quinolin-4,7-diamine,
- *N⁴*-(4-methoxyphenyl)-*N⁷*-(3-methoxyphenyl)quinolin-4,7-diamine,
- *N⁴*-(3-methoxyphenyl)-*N⁷*-(4-methoxyphenyl)quinolin-4,7-diamine,
- *N⁴*-(3,5-difluorophenyl)-*N⁷*-(3-methoxyphenyl)quinolin-4,7-diamine,
- *N*-(4-methoxyphenyl)-7-chloro-2-methoxy-quinolin-4-amine,
- *N*-(3,4-dimethoxyphenyl)-7-chloro-2-morpholino-quinolin-4-amine,
- *N*-(3,4-dimethoxyphenyl)-7-chloro-2-(4-methylpiperazin-1-yl)-quinolin-4-amine,
- *N*-(4-methoxyphenyl)-7-chloro-2-morpholino-quinolin-4-amine,
- *N*-(4-methoxyphenyl)-7-chloro-2-(4-methylpiperazin-1-yl)-quinolin-4-amine,
- *N*-(3,4-dimethoxyphenyl)-7-chloro-2-(2-morpholinoethoxy)quinolin-4-amine,
- *N*-(3,4-dimethoxyphenyl)-7-chloro-2-methoxy-quinolin-4-amine,
- 7-chloro-*N*-(2,3-dihydrobenzo[*b*][1,4]dioxin-6-yl)quinolin-4-amine, and
- 7-chloro-*N-*(benzo[d][1,3]dioxol-5-yl)quinolin-4-amine;
or a pharmaceutically acceptable salt and/or optical isomer, tautomer, solvate or isotopic variation thereof.

18. The compound for use according to any one of claims 15 to 17, wherein said compound is to be administered in combination with an antibody selected from an anti-PD1 antibody, an anti-CTLA4 antibody, an anti-PD-L1 antibody and a mixture of two or more thereof.

## Patentansprüche

1. Eine Verbindung der allgemeinen Formel (I): 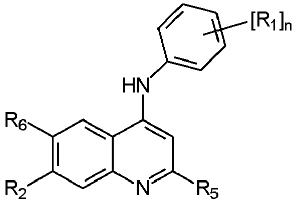 wobei
R₁ ausgewählt ist aus H, Alkyl, Halogen, OH, O-Alkyl, NH₂, NH-Alkyl, N-(Alkyl)₂, S-Alkyl, CF₃, OCF₃, OCF₂H und O(CH₂)ₙ₁O(CH₂)ₙ₂CH₃, wobei n₁ gleich 1 bis 4 ist und n₂ gleich 0 bis 3 ist,
Halogen ausgewählt ist aus Cl, F, Br und I;
n gleich 1 oder 2 ist,
wenn n gleich 2 ist, R₁ auch mit der Phenylgruppe in meta- und para-Stellung eine Dioxolangruppe oder eine 1,4-Dioxangruppe bilden kann,
R₂ ausgewählt ist aus Halogen und NR₃R₄, wobei Halogen wie vorstehend definiert ist,
R₃ für H steht und R₄ Phenyl ist, welches gegebenenfalls mit einem oder mehreren Substituenten, ausgewählt aus OH, O-Alkyl und Halogen, substituiert ist,
oder R₃ und R₄ zusammen eine (CH₂)₂O(CH₂)₂-Kette bilden, das heißt, dass NR₃R₄ eine Morpholinogruppe 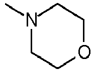 bildet,
R₅ ausgewählt ist aus H, einem cyclischen Rest, O-Alkyl, O-(CH₂)ₙ₁-(cyclischer Rest), (CH₂)ₙ₁-(cyclischer Rest), wobei n₁ wie vorstehend definiert ist, wobei der cyclische Rest ausgewählt ist aus 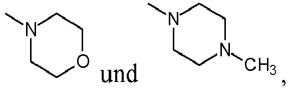
R₆ ausgewählt ist aus H und Halogen,
mit der Maßgabe, dass, wenn R₂ für Halogen steht oder R₆ für Halogen steht, R₅ nicht H ist,
oder ein pharmazeutisch verträgliches Salz und/oder optisches Isomer, Tautomer, Solvat oder eine isotopische Variation davon.

2. Eine Verbindung gemäß Anspruch 1 der allgemeinen Formel (2): 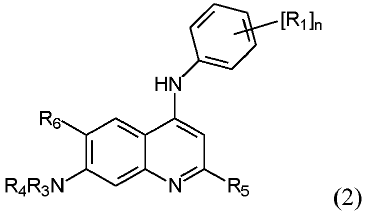
wobei R₁, R₃, R₄, R₅, R₆ und n wie in Anspruch 1 definiert sind,
oder ein pharmazeutisch verträgliches Salz und/oder optisches Isomer, Tautomer, Solvat oder eine isotopische Variation davon.

3. Eine Verbindung gemäß Anspruch 1 der allgemeinen Formel (3): 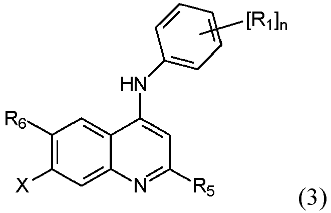
wobei R₁, R₅, R₆ und n wie in Anspruch 1 definiert sind und X für Halogen, ausgewählt aus Cl, F, Br und I, steht, und mit der Maßgabe, dass R₅ nicht H ist,
oder ein pharmazeutisch verträgliches Salz und/oder optisches Isomer, Tautomer, Solvat oder eine isotopische Variation davon.

4. Eine Verbindung gemäß einem der Ansprüche 1 bis 3, wobei R₁ ausgewählt ist aus Methyl (CH₃), Fluor (F), Chlor (Cl), Hydroxy (OH), Methoxy (OCH₃), CF₃, OCF₃, OCF₂H und O(CH₂)₂OCH₃, wobei, wenn n gleich 2 ist, die Substituenten R₁ gleich oder verschieden sind.

5. Eine Verbindung gemäß einem der Ansprüche 1 bis 3, wobei, wenn n gleich 2 ist, die beiden R₁ mit der Phenylgruppe in meta- und para-Stellung eine Dioxolangruppe oder eine 1,4-Dioxangruppe bilden.

6. Eine Verbindung gemäß einem der Ansprüche 1, 2 und 4, wobei R₃ für H steht und R₄ für Phenyl steht, welches unsubstituiert oder mit einer oder zwei Gruppen substituiert ist, welche gleich oder verschieden sind, ausgewählt aus OH, OCH₃ und Halogen, vorzugsweise Chlor.

7. Eine Verbindung gemäß einem der Ansprüche 1, 2 und 4, wobei R₃ und R₄ zusammen eine (CH₂)₂O(CH₂)₂-Kette bilden, das heißt, dass NR₃R₄ eine Morpholinogruppe bildet.

8. Eine Verbindung gemäß Anspruch 1, welche ausgewählt ist aus:
- *N*-(3,5-Difluorphenyl)-7-morpholinochinolin-4-amin,
- *N*-(*p*-Tolyl)-7-morpholinochinolin-4-amin,
- *N*-(*o*-Tolyl)-7-morpholinochinolin-4-amin,
- *N*-(4-Methoxyphenyl)-7-morpholinochinolin-4-amin,
- *N*-(3-Methoxyphenyl)-7-morpholinochinolin-4-amin,
- *N*-(3-(Trifluormethyl)phenyl)-7-morpholinochinolin-4-amin,
- *N⁴*, *N*⁷-Bis(4-methoxyphenyl)chinolin-4,7-diamin,
- *N⁴*, *N⁷*-Bis(3-methoxyphenyl)chinolin-4,7-diamin,
- *N⁴*-(3,5-Difluorphenyl)-*N⁷*-(4-methoxyphenyl)chinolin-4,7-diamin,
-*N⁴*-(3,5-Difluorphenyl)-*N⁷*-(3-chlor-4-hydroxyphenyl)chinolin-4,7-diamin,
- *N⁴*-(*p*-Tolyl)-*N⁷*-(3-methoxyphenyl)chinolin-4,7-diamin,
- *N⁴*-(*p*-Tolyl)-*N⁷*-(4-methoxyphenyl)chinolin-4,7-diamin,
- *N⁴*-(3-(Trifluormethyl)phenyl)-*N⁷*-(4-methoxyphenyl)chinolin-4,7-diamin,
- *N⁴*-(*o*-Tolyl)-*N⁷*-(3-methoxyphenyl)chinolin-4,7-diamin,
- *N⁴*-(4-Methoxyphenyl)-*N⁷*-(3-methoxyphenyl)chinolin-4,7-diamin,
- *N⁴*-(3-Methoxyphenyl)-*N⁷*-(4-methoxyphenyl)chinolin-4,7-diamin,
- *N⁴*-(3,5-Difluorphenyl)-*N⁷*-(3-methoxyphenyl)chinolin-4,7-diamin,
- *N*-(4-Methoxyphenyl)-7-chlor-2-methoxy-chinolin-4-amin,
- *N*-(3,4-Dimethoxyphenyl)-7-chlor-2-morpholino-chinolin-4-amin,
- *N*-(3,4-Dimethoxyphenyl)-7-chlor-2-(4-methylpiperazin-1-yl)-chinolin-4-amin,
- *N*-(4-Methoxyphenyl)-7-chlor-2-morpholino-chinolin-4-amin,
-*N*-(4-Methoxyphenyl)-7-chlor-2-(4-methylpiperazin-1-yl)-chinolin-4-amin,
-*N*-(3,4-Dimethoxyphenyl)-7-chlor-2-(2-morpholinoethoxy)chinolin-4-amin und
- *N*-(3,4-Dimethoxyphenyl)-7-chlor-2-methoxy-chinolin-4-amin;
oder ein pharmazeutisch verträgliches Salz und/oder optisches Isomer, Tautomer, Solvat oder eine isotopische Variation davon.

9. Eine Verbindung gemäß einem der Ansprüche 1 bis 8 zur Verwendung bei der Behandlung von Krebs, wobei der Krebs vorzugsweise ausgewählt ist aus einem Melanom, Blasen-, Nieren-, Prostata-, Darm-, Lungen-, Brust- und Blutkrebs.

10. Ein Arzneimittel als ein Kombinationspräparat zur gleichzeitigen, gesonderten oder aufeinanderfolgenden Verwendung bei der Behandlung von Krebs, umfassend:
- eine Verbindung der allgemeinen Formel (I) 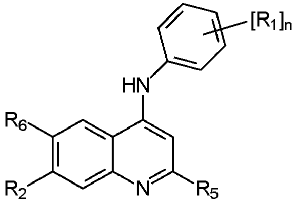 wobei
R₁ ausgewählt ist aus H, Alkyl, Halogen, OH, O-Alkyl, NH₂, NH-Alkyl, N-(Alkyl)₂, S-Alkyl, CF₃, OCF₃, OCF₂H und O(CH₂)ₙ₁O(CH₂)ₙ₂CH₃, wobei n₁ gleich 1 bis 4 ist und n₂ gleich 0 bis 3 ist,
Halogen ausgewählt ist aus Cl, F, Br und I;
n gleich 1 oder 2 ist;
wenn n gleich 2 ist, R₁ auch mit der Phenylgruppe in meta- und para-Stellung eine Dioxolangruppe oder eine 1,4-Dioxangruppe bilden kann,
R₂ ausgewählt ist aus H, Halogen und NR₃R₄; wobei Halogen wie vorstehend definiert ist,
R₃ für H steht und R₄ Phenyl ist, welches gegebenenfalls mit einem oder mehreren Substituenten, ausgewählt aus OH, O-Alkyl und Halogen, substituiert ist, oder R₃ und R₄ zusammen eine (CH₂)₂O(CH₂)₂-Kette bilden,
R₅ ausgewählt ist aus H, einem cyclischen Rest, O-Alkyl, O-(CH₂)ₙ₁-(cyclischer Rest), (CH₂)ₙ₁-(cyclischer Rest), n₁ wie vorstehend definiert ist, wobei der cyclische Rest ausgewählt ist aus 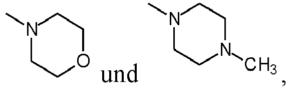
R₆ ausgewählt ist aus H und Halogen,
oder ein pharmazeutisch verträgliches Salz und/oder optisches Isomer, Tautomer, Solvat oder eine isotopische Variation davon,
- mindestens einen Antikörper, ausgewählt aus einem Anti-PD1-Antikörper, einem Anti-CTLA4-Antikörper, einem Anti-PD-L1-Antikörper und einem Gemisch aus zwei oder mehreren davon, und
- gegebenenfalls mindestens einen pharmazeutisch verträglichen Träger.

11. Das Arzneimittel zur Verwendung gemäß Anspruch 10, wobei die Verbindung (I) ausgewählt ist aus:
- *N*-(3,5 -Difluorphenyl)-7-chlorchinolin-4-amin,
- *N*-(4-Methylphenyl)-7-chlorchinolin-4-amin,
- *N*-(3-Trifluormethylphenyl)-7-chlorchinolin-4-amin,
- *N*-(2-Methylphenyl)-7-chlorchinolin-4-amin,
- *N*-(4-Methoxyphenyl)-7-chlorchinolin-4-amin,
- *N*-(3-Methoxyphenyl)-7-chlorchinolin-4-amin,
- *N*-(4-Hydroxyphenyl)-7-chlorchinolin-4-amin,
- *N*-(4-Hydroxy-3-chlorphenyl)-7-chlorchinolin-4-amin,
- *N*-(4-Trifluormethylphenyl)-7-chlorchinolin-4-amin,
- *N*-(3,5-Difluorphenyl)-7-morpholinochinolin-4-amin,
- *N*-(4-(Difluormethoxy)phenyl)-7-chlor-chinolin-4-amin,
- *N*-(4-Methoxy-3-(2-methoxyethoxy)phenyl)-7-chlor-chinolin-4-amin,
- *N* (4-Trifluormethoxyphenyl)-6-chlorchinolin-4-amin,
- *N*-(2,4-Dimethoxyphenyl)-6-chlorchinolin-4-amin,
- *N*-(3,4-Dimethoxyphenyl)-7-chlorchinolin-4-amin,
- *N*-(2,4-Dimethoxyphenyl)-7-chlorchinolin-4-amin,
- *N* (*p*-Tolyl)-7-morpholinochinolin-4-amin,
- *N*-(*o*-Tolyl)-7-morpholinochinolin-4-amin,
- *N*-(4-Methoxyphenyl)-7-morpholinochinolin-4-amin,
- *N*-(3-Methoxyphenyl)-7-morpholinochinolin-4-amin,
- *N*-(3-(Trifluormethyl)phenyl)-7-morpholinochinolin-4-amin,
- *N⁴*, *N⁷*-Bis(4-methoxyphenyl)chinolin-4,7-diamin,
- *N⁴*, *N⁷*-Bis(3-methoxyphenyl)chinolin-4,7-diamin,
- *N⁴*-(3,5-Difluorphenyl)-*N⁷*-(4-methoxyphenyl)chinolin-4,7-diamin,
-*N⁴*-(3,5-Difluorphenyl)-*N⁷*-(3-chlor-4-hydroxyphenyl)chinolin-4,7-diamin,
- *N⁴*-(*p*-Tolyl)-*N⁷*-(3-methoxyphenyl)chinolin-4,7-diamin,
- *N⁴*-(*p*-Tolyl)-*N⁷*-(4-methoxyphenyl)chinolin-4,7-diamin,
- *N⁴*-(3-(Trifluormethyl)phenyl)-*N⁷*-(4-methoxyphenyl)chinolin-4,7-diamin,
- *N⁴*-(*o*-Tolyl)-*N⁷*-(3-methoxyphenyl)chinolin-4,7-diamin,
- *N⁴*-(4-Methoxyphenyl)-*N⁷*-(3-methoxyphenyl)chinolin-4,7-diamin,
- *N⁴*-(3-Methoxyphenyl)-*N⁷*-(4-methoxyphenyl)chinolin-4,7-diamin,
- *N⁴*-(3,5-Difluorphenyl)-*N⁷*-(3-methoxyphenyl)chinolin-4,7-diamin,
- *N*-(4-Methoxyphenyl)-7-chlor-2-methoxy-chinolin-4-amin,
- *N*-(3,4-Dimethoxyphenyl)-7-chlor-2-morpholino-chinolin-4-amin,
- *N-*(3,4-Dimethoxyphenyl)-7-chlor-2-(4-methylpiperazin-1-yl)-chinolin-4-amin,
- *N-*(4-Methoxyphenyl)-7-chlor-2-morpholino-chinolin-4-amin,
-*N-*(4-Methoxyphenyl)-7-chlor-2-(4-methylpiperazin-1-yl)-chinolin-4-amin,
- *N*-(3,4-Dimethoxyphenyl)-7-chlor-2-(2-morpholinoethoxy)chinolin-4-amin,
- *N*-(3,4-Dimethoxyphenyl)-7-chlor-2-methoxy-chinolin-4-amin,
- 7-Chlor-*N*-(2,3-dihydrobenzo[*b*][1,4]dioxin-6-yl)chinolin-4-amin, und
- 7-Chlor-*N*-(benzo[d][1,3]dioxol-5-yl)chinolin-4-amin,
oder ein pharmazeutisch verträgliches Salz und/oder optisches Isomer, Tautomer, Solvat oder eine isotopische Variation davon.

12. Ein Arzneimittel, umfassend eine Verbindung gemäß einem der Ansprüche 1 bis 8 und einen pharmazeutisch verträglichen Träger.

13. Das Arzneimittel gemäß Anspruch 12, welches zusätzlich ein anderes Krebsmedikament umfasst.

14. Das Arzneimittel zur Verwendung gemäß Anspruch 10 oder 11, wobei der Krebs ausgewählt ist aus einem Melanom, Blasen-, Nieren-, Prostata-, Darm-, Lungen-, Brust- und Blutkrebs.

15. Eine Verbindung der allgemeinen Formel (I): 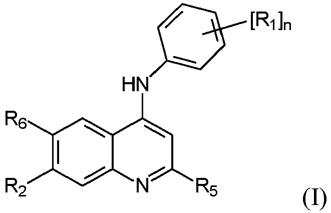 wobei
R₁ ausgewählt ist aus H, Halogen, OH, O-Alkyl, NH₂, NH-Alkyl, N-(Alkyl)₂, S-Alkyl, OCF₃, OCF₂H und O(CH₂)ₙ₁O(CH₂)ₙ₂CH₃, wobei n₁ gleich 1 bis 4 ist und n₂ gleich 0 bis 3 ist, wobei Halogen aus Cl, F, Br und I ausgewählt ist;
n gleich 1 oder 2 ist,
wenn n gleich 2 ist, R₁ auch mit der Phenylgruppe in meta- und para-Stellung eine Dioxolangruppe oder eine 1,4-Dioxangruppe bilden kann,
R₂ ausgewählt ist aus Halogen und NR₃R₄, wobei Halogen wie vorstehend definiert ist,
R₃ für H steht und R₄ Phenyl ist, welches gegebenenfalls mit einem oder mehreren Substituenten, ausgewählt aus OH, O-Alkyl und Halogen, substituiert ist,
oder R₃ und R₄ zusammen eine (CH₂)₂O(CH₂)₂-Kette bilden, das heißt, dass NR₃R₄ eine Morpholinogruppe 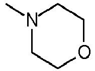 bildet,
R₅ ausgewählt ist aus H, einem cyclischen Rest, O-Alkyl, O-(CH₂)ₙ₁-(cyclischer Rest), (CH₂)ₙ₁-(cyclischer Rest), wobei n₁ wie vorstehend definiert ist, wobei der cyclische Rest ausgewählt ist aus 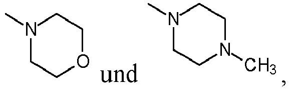
R₆ ausgewählt ist aus H und Halogen,
mit der Maßgabe, dass, wenn R₂ für F steht und R₅ = R₆ = H, R₁ nicht 3-Cl; 4-Cl; 3-F; 4-OCH₃; 3-Cl und 4-F; 3-Cl und 4-Cl; oder 4-F ist;
und mit der Maßgabe, dass, wenn R₂ für Cl steht und R₅ = R₆ = H, R₁ nicht 4-OH oder 4-Cl ist;
oder ein pharmazeutisch verträgliches Salz und/oder optisches Isomer, Tautomer, Solvat oder eine isotopische Variation davon,
zur Verwendung bei der Behandlung von Krebs.

16. Die Verbindung zur Verwendung gemäß Anspruch 15, wobei der Krebs ausgewählt ist aus einem Melanom, Blasen-, Nieren-, Prostata-, Darm-, Lungen-, Brust- und Blutkrebs, vorzugsweise wobei der Krebs ausgewählt ist aus einem Melanom und Darmkrebs.

17. Die Verbindung zur Verwendung gemäß Anspruch 15 oder 16, wobei die Verbindung ausgewählt ist aus:
- *N*-(3,5-Difluorphenyl)-7-chlorchinolin-4-amin,
- *N* (4-Methoxyphenyl)-7-chlorchinolin-4-amin,
- *N*-(3-Methoxyphenyl)-7-chlorchinolin-4-amin,
- *N*-(3,5-Difluorphenyl)-7-morpholinochinolin-4-amin,
- *N*-(4-(Difluormethoxy)phenyl)-7-chlor-chinolin-4-amin,
- *N-*(4-Methoxy-3-(2-methoxyethoxy)phenyl)-7-chlor-chinolin-4-amin,
- *N*-(3,4-Dimethoxyphenyl)-7-chlorchinolin-4-amin,
- *N*-(2,4-Dimethoxyphenyl)-7-chlorchinolin-4-amin,
- *N-*(4-Methoxyphenyl)-7-morpholinochinolin-4-amin,
- *N*-(3-Methoxyphenyl)-7-morpholinochinolin-4-amin,
- *N⁴*, *N⁷*-Bis(4-methoxyphenyl)chinolin-4,7-diamin,
- *N⁴*, *N⁷*-Bis(3-methoxyphenyl)chinolin-4,7-diamin,
- *N⁴*-(3,5-Difluorphenyl)-*N⁷*-(4-methoxyphenyl)chinolin-4,7-diamin,
-*N⁴*-(3,5-Difluorphenyl)-*N⁷*-(3-chlor-4-hydroxyphenyl)chinolin-4,7-diamin,
- *N⁴*-(4-Methoxyphenyl)-*N⁷*-(3-methoxyphenyl)chinolin-4,7-diamin,
- *N⁴*-(3-Methoxyphenyl)-*N⁷*-(4-methoxyphenyl)chinolin-4,7-diamin,
- *N⁴*-(3,5-Difluorphenyl)-*N⁷*-(3-methoxyphenyl)chinolin-4,7-diamin,
- *N-*(4-Methoxyphenyl)-7-chlor-2-methoxy-chinolin-4-amin,
- *N*-(3,4-Dimethoxyphenyl)-7-chlor-2-morpholino-chinolin-4-amin,
- *N*-(3,4-Dimethoxyphenyl)-7-chlor-2-(4-methylpiperazin-1-yl)-chinolin-4-amin,
- *N*-(4-Methoxyphenyl)-7-chlor-2-morpholino-chinolin-4-amin,
-*N*-(4-Methoxyphenyl)-7-chlor-2-(4-methylpiperazin-1-yl)-chinolin-4-amin,
- *N*-(3,4-Dimethoxyphenyl)-7-chlor-2-(2-morpholinoethoxy)chinolin-4-amin,
- *N*-(3,4-Dimethoxyphenyl)-7-chlor-2-methoxy-chinolin-4-amin,
- 7-Chlor-*N*-(2,3-dihydrobenzo[*b*][1,4]dioxin-6-yl)chinolin-4-amin, und
- 7-Chlor-*N*-(benzo[d][1,3]dioxol-5-yl)chinolin-4-amin;
oder ein pharmazeutisch verträgliches Salz und/oder optisches Isomer, Tautomer, Solvat oder eine isotopische Variation davon.

18. Die Verbindung zur Verwendung gemäß einem der Ansprüche 15 bis 17, wobei die Verbindung in Kombination mit einem Antikörper, ausgewählt aus einem Anti-PD1-Antikörper, einem Anti-CTLA4-Antikörper, einem Anti-PD-L1-Antikörper und einem Gemisch aus zwei oder mehreren davon, verabreicht werden soll.

## Revendications

1. Composé de formule générale (I) : dans laquelle 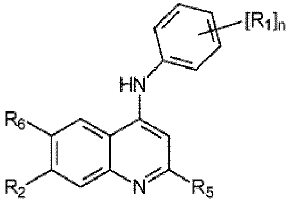
R₁ est sélectionné parmi H, alkyle, halogéno, OH, O-alkyle, NH₂, NH-alkyle, N-(alkyle)₂, S-alkyle, CF₃, OCF₃, OCF₂H et O(CH₂)ₙ₁O(CH₂)ₙ₂CH₃, n₁ valant de 1 à 4 et n₂ valant de 0 à 3, l'halogéno étant choisi parmi Cl, F, Br et I ;
n vaut 1 ou 2,
quand n vaut 2, alors il est aussi possible que R₁ forme, avec le groupe phényle aux positions méta et para, un groupe dioxolane ou un groupe 1,4-dioxane,
R₂ est sélectionné parmi halogéno et NR₃R₄, l'halogéno étant tel que défini précédemment,
R₃ est H et R₄ est un phényle optionnellement substitué par un ou plusieurs substituants sélectionnés parmi OH, O-alkyle et halogéno,
ou bien R₃ et R₄ forment ensemble une chaîne (CH₂)₂O(CH₂)₂, en d'autres termes NR₃R₄ forme un groupe morpholino 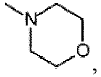
R₅ est sélectionné parmi H, un radical cyclique, O-alkyle, O-(CH₂)ₙ₁-(radical cyclique), (CH₂)ₙ₁-(radical cyclique), n₁ étant tel que défini précédemment, ledit radical cyclique étant choisi parmi 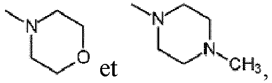
R₆ est sélectionné parmi H et halogéno,
sous réserve que, lorsque R₂ est un halogéno ou R₆ est un halogéno, alors R₅ ne soit pas H,
ou un sel et/ou isomère optique, tautomère, solvate ou variant isotopique, acceptable sur le plan pharmaceutique, d'un tel composé.

2. Composé selon la revendication 1, de formule générale (2) : 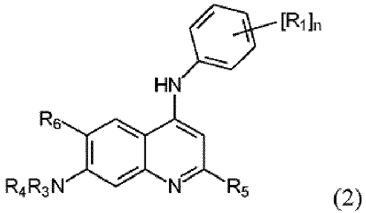
dans laquelle R₁, R₃, R₄, R₅, R₆ et n sont tels que définis dans la revendication 1,
ou un sel et/ou isomère optique, tautomère, solvate ou variant isotopique, acceptable sur le plan pharmaceutique, d'un tel composé.

3. Composé selon la revendication 1, de formule générale (3) : 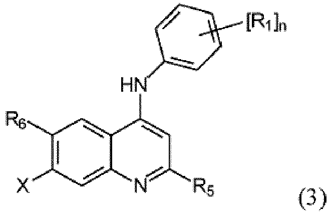
dans laquelle R₁, R₅, R₆ et n sont tels que définis dans la revendication 1 et X est un halogéno choisi parmi Cl, F, Br et I, et sous réserve que R₅ ne soit pas H,
ou un sel et/ou isomère optique, tautomère, solvate ou variant isotopique, acceptable sur le plan pharmaceutique, d'un tel composé.

4. Composé selon l'une quelconque des revendications 1 à 3, dans lequel R₁ est sélectionné parmi méthyle (CH₃), fluoro (F), chloro (Cl), hydroxy (OH), méthoxy (OCH₃), CF₃, OCF₃, OCF₂H et O(CH₂)₂OCH₃, et dans lequel, quand n vaut 2, alors les substituants R₁ sont identiques ou différents.

5. Composé selon l'une quelconque des revendications 1 à 3, dans lequel, quand n vaut 2, alors les deux R₁ forment, avec le groupe phényle aux positions méta et para, un groupe dioxolane ou un groupe 1,4-dioxane.

6. Composé selon l'une quelconque des revendications 1, 2 et 4, dans lequel R₃ est H et R₄ est un phényle non substitué ou substitué par un ou deux groupes identiques ou différents sélectionné parmi OH, OCH₃ et halogéno, de préférence chloro.

7. Composé selon l'une quelconque des revendications 1, 2 et 4, dans lequel R₃ et R₄ forment ensemble une chaîne (CH₂)₂O(CH₂)₂, en d'autres termes NR₃R₄ forme un groupe morpholino.

8. Composé selon la revendication 1, qui est sélectionné parmi :
- *N*-(3,5-difluorophényl)-7-morpholinoquinolin-4-amine,
- *N*-(*p*-tolyl)-7-morpholinoquinolin-4-amine,
- *N*-(*o*-tolyl)-7-morpholinoquinolin-4-amine,
- *N*-(4-méthoxyphényl)-7-morpholinoquinolin-4-amine,
- *N*-(3-méthoxyphényl)-7-morpholinoquinolin-4-amine,
- *N*-(3-(trifluorométhyl)phényl)-7-morpholinoquinolin-4-amine,
- *N*⁴,*N*⁷-bis(4-méthoxyphényl)quinolin-4,7-diamine,
- *N*⁴,*N*⁷-bis(3-méthoxyphényl)quinolin-4,7-diamine,
-*N*⁴-(3,5-difluorophényl)-*N*⁷-(4-méthoxyphényl)quinolin-4,7-diamine,
- *N*⁴-(3,5-difluorophényl)-*N*⁷-(3-chloro-4-hydroxyphényl)quinolin-4,7-diamine,
-*N*⁴-(*p*-tolyl)-*N*⁷-(3-méthoxyphényl)quinolin-4,7-diamine,
-*N*⁴-(*p*-tolyl)-*N*⁷-(4-méthoxyphényl)quinolin-4,7-diamine,
-*N*⁴-(3-(trifluorométhyl)phényl)-*N*⁷-(4-méthoxyphényl)quinolin-4,7-diamine,
- *N*⁴-(*o*-tolyl)-*N*⁷-(3-méthoxyphényl)quinolin-4,7-diamine,
-*N*⁴-(4-méthoxyphényl)-*N*⁷-(3-méthoxyphényl)quinolin-4,7-diamine,
-*N*⁴-(3-méthoxyphényl)-*N*⁷-(4-méthoxyphényl)quinolin-4,7-diamine,
-*N*⁴-(3,5-difluorophényl)-*N*⁷-(3-méthoxyphényl)quinolin-4,7-diamine,
- *N*-(4-méthoxyphényl)-7-chloro-2-méthoxyquinolin-4-amine,
- *N*-(3,4-diméthoxyphényl)-7-chloro-2-morpholinoquinolin-4-amine,
- *N*-(3,4-diméthoxyphényl)-7-chloro-2-(4-méthylpipérazin-1-yl)quinolin-4-amine,
- *N*-(4-méthoxyphényl)-7-chloro-2-morpholinoquinolin-4-amine,
- *N*-(4-méthoxyphényl)-7-chloro-2-(4-méthylpipérazin-1-yl)quinolin-4-amine,
- *N*-(3,4-diméthoxyphényl)-7-chloro-2-(2-morpholinoéthoxy)quinolin-4-amine et
- *N*-(3,4-diméthoxyphényl)-7-chloro-2-méthoxyquinolin-4-amine,
ou un sel et/ou isomère optique, tautomère, solvate ou variant isotopique, acceptable sur le plan pharmaceutique, d'un tel composé.

9. Composé selon l'une quelconque des revendications 1 à 8 pour une utilisation dans le traitement du cancer, ledit cancer étant de préférence sélectionné parmi un mélanome et les cancers de la vessie, des reins, de la prostate, du côlon, du poumon, du sein et du sang.

10. Composition pharmaceutique sous la forme d'une préparation combinée pour une utilisation simultanée, séparée ou séquentielle dans le traitement du cancer, comprenant :
- un composé de formule générale (I) dans laquelle 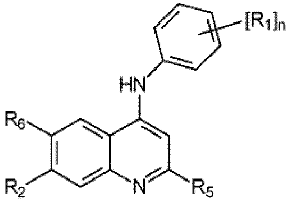
R₁ est sélectionné parmi H, alkyle, halogéno, OH, O-alkyle, NH₂, NH-alkyle, N-(alkyle)₂, S-alkyle, CF₃, OCF₃, OCF₂H et O(CH₂)ₙ₁O(CH₂)ₙ₂CH₃, n₁ valant de 1 à 4 et n₂ valant de 0 à 3, l'halogéno étant sélectionné parmi Cl, F, Br et I ;
n vaut 1 ou 2,
quand n vaut 2, alors il est aussi possible que R₁ forme, avec le groupe phényle aux positions méta et para, un groupe dioxolane ou un groupe 1,4-dioxane,
R₂ est sélectionné parmi H, halogéno et NR₃R₄, l'halogéno étant tel que défini précédemment,
R₃ est H et R₄ est un phényle éventuellement substitué par un ou plusieurs substituants sélectionnés parmi OH, O-alkyle et halogéno, ou bien R₃ et R₄ forment ensemble une chaîne (CH₂)₂O(CH₂)₂,
R₅ est sélectionné parmi H, un radical cyclique, O-alkyle, O-(CH₂)ₙ₁-(radical cyclique), (CH₂)ₙ₁-(radical cyclique), n₁ étant tel que défini précédemment, ledit radical cyclique étant choisi parmi 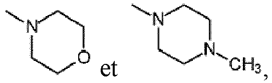
R₆ est sélectionné parmi H et halogéno,
ou un sel et/ou isomère optique, tautomère, solvate ou variant isotopique, acceptable sur le plan pharmaceutique, d'un tel composé,
- au moins un anticorps sélectionné parmi un anticorps anti-PD1, un anticorps anti-CTLA4, un anticorps anti-PD-L1 et un mélange de deux ou plus de ceux-ci, et
- optionnellement au moins un véhicule acceptable sur le plan pharmaceutique.

11. Composition pharmaceutique pour une utilisation selon la revendication 10, dans laquelle le composé (I) est sélectionné parmi:
- *N*-(3,5-difluorophényl)-7-chloroquinolin-4-amine,
- *N*-(4-méthylphényl)-7-chloroquinolin-4-amine,
- *N*-(3-trifluorométhylphényl)-7-chloroquinolin-4-amine,
- *N*-(2-méthylphényl)-7-chloroquinolin-4-amine,
- *N*-(4-méthoxyphényl)-7-chloroquinolin-4-amine,
- *N*-(3-méthoxyphényl)-7-chloroquinolin-4-amine,
- *N*-(4-hydroxyphényl)-7-chloroquinolin-4-amine,
- *N*-(4-hydroxy-3-chlorophényl)-7-chloroquinolin-4-amine,
- *N*-(4-trifluorométhylphényl)-7-chloroquinolin-4-amine,
- *N*-(3,5-difluorophényl)-7-morpholinoquinolin-4-amine,
- *N*-(4-(difluorométhoxy)phényl)-7-chloroquinolin-4-amine,
- *N*-(4-méthoxy-3-(2-méthoxyéthoxy)phényl)-7-chloroquinolin-4-amine,
- *N*-(4-trifluorométhoxyphényl)-6-chloroquinolin-4-amine,
- *N*-(2,4-diméthoxyphényl)-6-chloroquinolin-4-amine,
- *N*-(3,4-diméthoxyphényl)-7-chloroquinolin-4-amine,
- *N*-(2,4-diméthoxyphényl)-7-chloroquinolin-4-amine,
- *N*-(*p*-tolyl)7-morpholinoquinolin-4-amine,
- *N*-(*o*-tolyl)-7-morpholinoquinolin-4-amine,
- *N*-(4-méthoxyphényl)-7-morpholinoquinolin-4-amine,
- *N*-(3-méthoxyphényl)-7-morpholinoquinolin-4-amine,
- *N*-(3-(trifluorométhyl)phényl)-7-morpholinoquinolin-4-amine,
- *N*⁴,*N*⁷-bis(4-méthoxyphényl)quinolin-4,7-diamine,
- *N*⁴,*N*⁷-bis(3-méthoxyphényl)quinolin-4,7-diamine,
-*N*⁴-(3,5-difluorophényl)-*N*⁷-(4-méthoxyphényl)quinolin-4,7-diamine,
- *N*⁴-(3,5-difluorophényl)-*N*⁷-(3-chloro-4-hydroxyphényl)quinolin-4,7-diamine,
-*N*⁴-(*p*-tolyl)-*N*⁷-(3-méthoxyphényl)quinolin-4,7-diamine,
-*N*⁴-(*p*-tolyl)-*N*⁷-(4-méthoxyphényl)quinolin-4,7-diamine,
-*N*⁴-(3-(trifluorométhyl)phényl)-*N*⁷-(4-méthoxyphényl)quinolin-4,7-diamine,
- *N*⁴-(*o*-tolyl)-*N*⁷-(3-méthoxyphényl)quinolin-4,7-diamine,
-*N*⁴-(4-méthoxyphényl)-*N*⁷-(3-méthoxyphényl)quinolin-4,7-diamine,
-*N*⁴-(3-méthoxyphényl)-*N*⁷-(4-méthoxyphényl)quinolin-4,7-diamine,
-*N*⁴-(3,5-difluorophényl)-*N*⁷-(3-méthoxyphényl)quinolin-4,7-diamine,
- *N*-(4-méthoxyphényl)-7-chloro-2-méthoxyquinolin-4-amine,
- *N*-(3,4-diméthoxyphényl)-7-chloro-2-morpholinoquinolin-4-amine,
- *N*-(3,4-diméthoxyphényl)-7-chloro-2-(4-méthylpipérazin-1-yl)quinolin-4-amine,
- *N*-(4-méthoxyphényl)-7-chloro-2-morpholinoquinolin-4-amine,
- *N*-(4-méthoxyphényl)-7-chloro-2-(4-méthylpipérazin-1-yl)quinolin-4-amine,
- *N*-(3,4-diméthoxyphényl)-7-chloro-2-(2-morpholinoéthoxy)quinolin-4-amine,
- *N*-(3,4-diméthoxyphényl)-7-chloro-2-méthoxy-quinolin-4-amine,
- 7-chloro-*N*-(2,3-dihydrobenzo[b][1,4]dioxin-6-yl)quinolin-4-amine, et
- 7-chloro-*N*-(benzo[d][1,3]dioxol-5-yl)quinolin-4-amine ;
ou un sel et/ou isomère optique, tautomère, solvate ou variant isotopique, acceptable sur le plan pharmaceutique, d'un tel composé.

12. Composition pharmaceutique comprenant un composé selon l'une quelconque des revendications 1 à 8 et un véhicule acceptable sur le plan pharmaceutique.

13. Composition pharmaceutique selon la revendication 12, comprenant de plus un autre médicament anticancéreux.

14. Composition pharmaceutique pour une utilisation selon la revendication 10 ou 11, dans laquelle ledit cancer est sélectionné parmi un mélanome et les cancers de la vessie, des reins, de la prostate, du côlon, du poumon, du sein et du sang.

15. Composé de formule générale (I) : dans laquelle 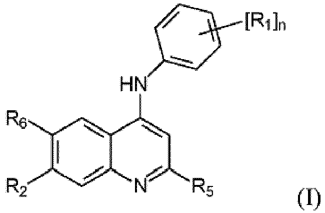
R₁ est sélectionné parmi H, halogéno, OH, O-alkyle, NH₂, NH-alkyle, N-(alkyle)₂, S-alkyle, OCF₃, OCF₂H et O(CH₂)ₙ₁O(CH₂)ₙ₂CH₃, n₁ valant de 1 à 4 et n₂ valant de 0 à 3, l'halogéno étant sélectionné parmi Cl, F, Br et I ;
n vaut 1 ou 2,
quand n vaut 2, alors il est aussi possible que R₁ forme, avec le groupe phényle aux positions méta et para, un groupe dioxolane ou un groupe 1,4-dioxane,
R₂ est sélectionné parmi halogéno et NR₃R₄, l'halogéno étant tel que défini précédemment,
R₃ est H et R₄ est un phényle optionnellement substitué par un ou plusieurs substituants sélectionnés parmi OH, O-alkyle et halogéno,
ou bien R₃ et R₄ forment ensemble une chaîne (CH₂)₂O(CH₂)₂, en d'autres termes NR₃R₄ forme un groupe morpholino 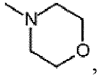
R₅ est sélectionné parmi H, un radical cyclique, O-alkyle, O-(CH₂)ₙ₁-(radical cyclique), (CH₂)ₙ₁-(radical cyclique), n₁ étant tel que défini précédemment, ledit radical cyclique étant choisi parmi 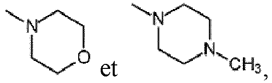
R₆ est sélectionné parmi H et halogéno,
sous réserve que, lorsque R₂ est F et R₅ = R₆ = H, alors R₁ ne soit pas 3-Cl ; 4-Cl ; 3-F ; 4-OCH₃ ; 3-Cl et 4-F ; 3-Cl et 4-Cl ; ou 4-F ;
et sous réserve que, lorsque R₂ est Cl et R₅ = R₆ = H, alors R₁ ne soit pas 4-OH ou 4-Cl ;
ou un sel et/ou isomère optique, tautomère, solvate ou variant isotopique, acceptable sur le plan pharmaceutique, d'un tel composé,
pour une utilisation dans le traitement du cancer.

16. Composé pour une utilisation selon la revendication 15, dans laquelle ledit cancer est sélectionné parmi un mélanome et les cancers de la vessie, des reins, de la prostate, du côlon, du poumon, du sein et du sang, de préférence dans lequel ledit cancer est sélectionné parmi un mélanome et un cancer du côlon.

17. Composé pour une utilisation selon la revendication 15 ou 16, lequel composé est sélectionné parmi :
- *N*-(3,5-difluorophényl)-7-chloroquinolin-4-amine,
- *N*-(4-méthoxyphényl)-7-chloroquinolin-4-amine,
- *N*-(3-méthoxyphényl)-7-chloroquinolin-4-amine,
- *N*-(3,5-difluorophényl)-7-morpholinoquinolin-4-amine,
- *N*-(4-(difluorométhoxy)phényl)-7-chloroquinolin-4-amine,
- *N*-(4-méthoxy-3-(2-méthoxyéthoxy)phényl)-7-chloroquinolin-4-amine,
- *N*-(3,4-diméthoxyphényl)-7-chloroquinolin-4-amine,
- *N*-(2,4-diméthoxyphényl)-7-chloroquinolin-4-amine,
- *N*-(4-méthoxyphényl)-7-morpholinoquinolin-4-amine,
- *N*-(3-méthoxyphényl)-7-morpholinoquinolin-4-amine,
- *N*⁴,*N*⁷-bis(4-méthoxyphényl)quinolin-4,7-diamine,
- *N*⁴,*N*⁷-bis(3-méthoxyphényl)quinolin-4,7-diamine,
-*N*⁴-(3,5-difluorophényl)-*N*⁷-(4-méthoxyphényl)quinolin-4,7-diamine,
- *N*⁴-(3,5-difluorophényl)-*N*⁷-(3-chloro-4-hydroxyphényl)quinolin-4,7-diamine,
-*N*⁴-(4-méthoxyphényl)-*N*⁷-(3-méthoxyphényl)quinolin-4,7-diamine,
-*N*⁴-(3-méthoxyphényl)-*N*⁷-(4-méthoxyphényl)quinolin-4,7-diamine,
-*N*⁴-(3,5-difluorophényl)-*N*⁷-(3-méthoxyphényl)quinolin-4,7-diamine,
- *N*-(4-méthoxyphényl)-7-chloro-2-méthoxyquinolin-4-amine,
- *N*-(3,4-diméthoxyphényl)-7-chloro-2-morpholinoquinolin-4-amine,
- *N*-(3,4-diméthoxyphényl)-7-chloro-2-(4-méthylpipérazin-1-yl)quinolin-4-amine,
- *N*-(4-méthoxyphényl)-7-chloro-2-morpholinoquinolin-4-amine,
- *N*-(4-méthoxyphényl)-7-chloro-2-(4-méthylpipérazin-1-yl)quinolin-4-amine,
- *N*-(3,4-diméthoxyphényl)-7-chloro-2-(2-morpholinoéthoxy)quinolin-4-amine,
- *N*-(3,4-diméthoxyphényl)-7-chloro-2-méthoxy-quinolin-4-amine,
- 7-chloro-N-(2,3-dihydrobenzo[b][1,4]dioxin-6-yl)quinolin-4-amine, et
- 7-chloro-N-(benzo[d][1,3]dioxol-5-yl)quinolin-4-amine ;
ou un sel et/ou isomère optique, tautomère, solvate ou variant isotopique, acceptable sur le plan pharmaceutique, d'un tel composé.

18. Composé pour une utilisation selon l'une quelconque des revendications 15 à 17, lequel composé est destiné à être administré en combinaison avec un anticorps sélectionné parmi un anticorps anti-PD1, un anticorps anti-CTLA4, un anticorps anti-PD-L1 et un mélange de deux ou plusieurs de ceux-ci.
